# EUROPEAN PATENT SPECIFICATION

(11) **EP 1 095 275 B1**
(45) Date of publication and mention of the grant of the patent: **27.05.2009**
(21) Application number: 99941990.6
(22) Date of filing: 30.07.1999
(51) Int. Cl.: G01N 33/68

(54) **ENDOGENOUS CONSTITUTIVELY ACTIVATED G PROTEIN-COUPLED ORPHAN RECEPTORS**
ENDOGENE, KONSTITUTIV AKTIVIERTE G-PROTEIN GEKOPPELTE WAISENREZEPTOREN
RECEPTEURS ORPHELINS ENDOGENES A ACTIVATION CONSTITUTIVE COUPLES A LA PROTEINE G

(30) Priority: 31.07.1998 US 94879 P; 30.10.1998 US 106300 P; 04.12.1998 US 110906 P; 26.02.1999 US 121851 P
(43) Date of publication of application: 02.05.2001
(73) Proprietor: Arena Pharmaceuticals, Inc., San Diego, CA 92121 (US)
(72) Inventor: BEHAN, Dominic, P., San Diego, CA (US); CHALMERS, Derek, T., Solana Beach, CA 92075 (US); LIAW, Chen, San Diego, CA 92129 (US); LIN, I-Lin, San Diego, CA 92126 (US); LOWITZ, Kevin, San Diego, CA 92108 (US); CHEN, Ruoping, San Diego, CA 92130 (US)
(74) Representative: Brasnett, Adrian Hugh
(86) International application number: PCT/US1999/017425
(87) International publication number: WO 2000/006597

(56) References cited:
- WO-A-97/11159
- WO-A-98/46995
- BERTIN B. ET AL.: "Cellular signaling by an agonist-activated receptor/Gs-alpha fusion protein" PROC. NATL. ACAD. SCI. USA, vol. 91, September 1994 (1994-09), pages 8827-8831, XP002128968
- SEIFERT R. ET AL.: "Different effects of Gs alpha splice variants on beta-2-adrenoreceptor-mediated signaling" J. BIOL. CHEM., vol. 273, no. 9, 27 February 1998 (1998-02-27), pages 5109-5116, XP002128969
- WISE A. ET AL.: "Rescue of functional interactions between the alpha-2A-adrenoreceptor and acylation-resistant forms of Gi1-alpha by expressing the proteins from chimeric open reading frames" J. BIOL. CHEM., vol. 272, no. 39, 26 September 1997 (1997-09-26), pages 24673-24678, XP002128970
- BURT A.R. ET AL.: "Agonist occupationof an alpha-2A-adrenoreceptor-Gi1-alpha fusion protein results in activation of both receptor-linked and endogenous Gi proteins" J. BIOL. CHEM., vol. 273, no. 17, 24 April 1997 (1997-04-24), pages 10367-10375, XP002128971

### SEQUENCE LISTING

(1) GENERAL INFORMATION:
   (i) APPLICANT: Behan, Dominic P.
      Chalmers, Derek T.
      Liaw, Chen
      Lin, I-Lin
      Lowitz, Kevin P.
      Chen, Ruoping
   (ii) TITLE OF INVENTION: Endogenous, Constitutively Activated
      G Protein-Coupled Orphan Receptors
   (iii) NUMBER OF SEQUENCES: 45
   (iv) CORRESPONDENCE ADDRESS:
      (A) ADDRESSEE:
      (B) STREET:
      (C) CITY:
      (D) STATE:
      (E) COUNTRY:
      (F) ZIP:
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER: US
      (B) FILING DATE:
      (C) CLASSIFICATION:
   (viii) ATTORNEY/AGENT INFORMATION:
      (A) NAME: Michael P. Straher
      (B) REGISTRATION NUMBER: 38,325
   (ix) TELECOMMUNICATION INFORMATION:
      (A) TELEPHONE:
      (B) TELEFAX:
(2) INFORMATION FOR SEQ ID NO:1:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:1:
      GGAGGATCCATGGCCTGGTTCTCAGC 26
(3) INFORMATION FOR SEQ ID NO:2:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:2:
      CACAAGCTTAGRCCRTCCMGRCARTTCCA 29
(4) INFORMATION FOR SEQ ID NO:3:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:3:
      GGAGAAGCTTCTGGCGGCGATGAACGCTAG 30
(5) INFORMATION FOR SEQ ID NO:4:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:4:
      ACAGGATCCAGGTGGCTGCTAGCAAGAG 28
(6) INFORMATION FOR SEQ ID NO:5:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:5:
      CTTAAGCTTAAAATGAACGAAGACCCGAAG 30
(7) INFORMATION FOR SEQ ID NO:6:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:6:
      GGAGGATCCCCAGAGCATCACTRGCAT 27
(8) INFORMATION FOR SEQ ID NO:7:
   (i) SEQUENCE CHARACTERISTICS:
      (A) LENGTH: 530 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:7:
   GGAGGATCCA TGGCCTGGTT CTCAGCCGGC TCAGGCAGTG TGAATGTGAG CATAGACCCA 60
   GCAGAGGAAC CTACAGGCCC AGCTACACTG CTGCCCTCTC CCAGGGCCTG GGATGTGGTG 120
   CTGTGCATCT CAGGCACCCT GGTGTCCTGC GAGAATGCTC TGGTGATGGC CATCATTGTG 180
   GGCACGCCTG CCTTCCGCGC CCCCATGTTC CTGCTGGTGG GCAGCTTGGC CGTAGCAGAC 240
   CTGCTGGCAG GCCTGGGCCT GGTCCTGCAC TTCGCTGCTG ACTTCTGTAT TGGCTCACCA 300
   GAGATGAGCT TGGTGCTGGT TGGCGTGCTA GCAACGGCCT TTACTGCCAG CATCGGCAGC 360
   CTGCTGGCCA TCACCGTTGA CCGCTACCTT TCCCTGTACA ACGCCCTCAC CTACTACTCA 420
   GAGACAACAG TAACTCGAAC CTACGTGATG CTGGCCTTGG TGTGGGTGGG TGCCCTGGGC 480
   CTGGGGCTGG TTCCCGTGCT GGCCTGGAAC TGCCGGGACG GTCTAAGCTT 530
(9) INFORMATION FOR SEQ ID NO:8:
   (i) SEQUENCE CHARACTERISTICS:
      (A) LENGTH: 601 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:8:
   AAGCTTCTGG CGGCGATGAA CGCTAGCGCC GCCGCGCTCA ACGAGTCCCA GGTGGTGGCA    60
   GTAGCGGCCG AGGGAGCGGC AGCTGCGGCT ACAGCAGCAG GGACACCGGA CACCAGCGAA   120
   TGGGGACCTC CGGCAGCATC CGCGGCGCTG GGAGGCGGCG GAGGACCTAA CGGGTCACTG   180
   GAGCTGTCTT CGCAGCTGCC CGCAGGACCC TCAGGACTTC TGCTTTCGGC AGTGAATCCC   240
   TGGGATGTGC TGCTGTGCGT GTCGGGGACT GTGATCGCAG GCGAAAATGC GCTGGTGGTG   300
   GCGCTCATCG CATCCACTCC CGCGCTGCGC ACGCCCATGT TTGTGCTCGT GGGTAGTCTG   360
   GCCACTGCTG ACCTGCTGGC GGGCTGTGGC CTCATCCTAC ACTTCGTGTT CCAGTACGTG   420
   GTGCCCTCGG AGACTGTGAG CCTGCTCATG GTGGGCTTCC TGGTGGCGTC CTTCGCCGCC   480
   TCAGTCAGCA GCCTGCTCGC TATCACAGTG GACCGTTACC TGTCCCTTTA CAACGCGCTC   540
   ACCTACTACT CGCGCCGGAC CCTGTTGGGC GTGCACCTCT TGCTAGCAGC CACCTGGATC   600
   C                                                                   601
(10) INFORMATION FOR SEQ ID NO:9:
   (i) SEQUENCE CHARACTERISTICS:
      (A) LENGTH: 510 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:9:
   AAGCTTAAAA TGAACGAAGA CCCGAAGGTC AATTTAAGCG GGCTGCCTCG GGACTGTATA 60
   GAAGCTGGTA CTCCGGAGAA CATCTCAGCC GCTGTCCCCT CCCAGGGCTC TGTTGTGGAG 120
   TCAGAACCCG AGCTCGTTGT CAACCCCTGG GACATTGTCT TGTGCAGCTC AGGAACCCTC 180
   ATCTGCTGTG AAAATGCCGT CGTGGTCCTT ATCATCTTCC ACAGCCCCAG CCTGCGAGCA 240
   CCCATGTTCC TGCTGATAGG CAGCCTGGCT CTTGCAGACC TGCTGGCTGG TCTGGGACTC 300
   ATCATCAATT TTGTTTTTGC CTACCTGCTT CAGTCAGAAG CCACCAAGCT GGTCACAATT 360
   GGACTCATTG TCGCCTCTTT CTCTGCCTCT GTCTGCAGTT TGCTGGCTAT CACTGTGGAC 420
   CGCTACCTCT CGCTGTATTA CGCCCTGACG TACCACTCCG AGAGGACCGT CACCTTTACC 480
   TATGTCATGC TAGTGATGCT CTGGGGATCC 510
(11) INFORMATION FOR SEQ ID NO:10:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:10:
      CTTAAGCTTGTGGCATTTGGTACT 24
(12) INFORMATION FOR SEQ ID NO:11:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:11:
      TCTGGATCCTTGGCCAGGCAGTGGAAGT 28
(13) INFORMATION FOR SEQ ID NO:12:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:12:
      GAGAATTCAC TCCTGAGCTC AAGATGAACT 30
(14) INFORMATION FOR SEQ ID NO:13:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:13:
      CGGGATCCCC GTAACTGAGC CACTTCAGAT 30
(15) INFORMATION FOR SEQ ID NO:14:
   (i) SEQUENCE CHARACTERISTICS:
      (A) LENGTH: 1050 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:14:
   ATGAACTCCA CCTTGGATGG TAATCAGAGC AGCCACCCTT TTTGCCTCTT GGCATTTGGC 60
   TATTTGGAAA CTGTCAATTT TTGCCTTTTG GAAGTATTGA TTATTGTCTT TCTAACTGTA 120
   TTGATTATTT CTGGCAACAT CATTGTGATT TTTGTATTTC ACTGTGCACC TTTGTTGAAC 180
   CATCACACTA CAAGTTATTT TATCCAGACT ATGGCATATG CTGACCTTTT TGTTGGGGTG 240
   AGCTGCGTGG TCCCTTCTTT ATCACTCCTC CATCACCCCC TTCCAGTAGA GGAGTCCTTG 300
   ACTTGCCAGA TATTTGGTTT TGTAGTATCA GTTCTGAAGA GCGTCTCCAT GGCTTCTCTG 360
   GCCTGTATCA GCATTGATAG ATACATTGCC ATTACTAAAC CTTTAACCTA TAATACTCTG 420
   GTTACACCCT GGAGACTACG CCTGTGTATT TTCCTGATTT GGCTATACTC GACCCTGGTC 480
   TTCCTGCCTT CCTTTTTCCA CTGGGGCAAA CCTGGATATC ATGGAGATGT GTTTCAGTGG 540
   TGTGCGGAGT CCTGGCACAC CGACTCCTAC TTCACCCTGT TCATCGTGAT GATGTTATAT 600
   GCCCCAGCAG CCCTTATTGT CTGCTTCACC TATTTCAACA TCTTCCGCAT CTGCCAACAG 660
   CACACAAAGG ATATCAGCGA AAGGCAAGCC CGCTTCAGCA GCCAGAGTGG GGAGACTGGG 720
   GAAGTGCAGG CCTGTCCTGA TAAGCGCTAT GCCATGGTCC TGTTTCGAAT CACTAGTGTA 780
   TTTTACATCC TCTGGTTGCC ATATATCATC TACTTCTTGT TGGAAAGCTC CACTGGCCAC 840
   AGCAACCGCT TCGCATCCTT CTTGACCACC TGGCTTGCTA TTAGTAACAG TTTCTGCAAC 900
   TGTGTAATTT ATAGTCTCTC CAACAGTGTA TTCCAAAGAG GACTAAAGCG CCTCTCAGGG 960
   GCTATGTGTA CTTCTTGTGC AAGTCAGACT ACAGCCAACG ACCCTTACAC AGTTAGAAGC 1020
   AAAGGCCCTC TTAATGGATG TCATATCTGA 1050
(16) INFORMATION FOR SEQ ID NO:15:
   (i) SEQUENCE CHARACTERISTICS:
      (A) LENGTH: 349 amino acids
      (B) TYPE: amino acid
      (C) STRANDEDNESS:
      (D) TOPOLOGY: not relevant
   (ii) MOLECULE TYPE: protein
   (xi) SEQUENCE DESCRIPTION: SEQ ID NO:15:
   Met Asn Ser Thr Leu Asp Gly Asn Gln Ser Ser His Pro Phe Cys Leu
   1               5                   10                  15
   Leu Ala Phe Gly Tyr Leu Glu Thr Val Asn Phe Cys Leu Leu Glu Val
               20                  25                  30
   Leu Ile Ile Val Phe Leu Thr Val Leu Ile Ile Ser Gly Asn Ile Ile
           35                  40                  45
   Val Ile Phe Val Phe His Cys Ala Pro Leu Leu Asn His His Thr Thr
       50                  55                  60
   Ser Tyr Phe Ile Gln Thr Met Ala Tyr Ala Asp Leu Phe Val Gly Val
   65                  70                  75                  80
   Ser Cys Val Val Pro Ser Leu Ser Leu Leu His His Pro Leu Pro Val
                   85                  90                  95
   Glu Glu Ser Leu Thr Cys Gln Ile Phe Gly Phe Val Val Ser Val Leu
               100                 105                 110
   Lys Ser Val Ser Met Ala Ser Leu Ala Cys Ile Ser Ile Asp Arg Tyr
           115                 120                 125
   Ile Ala Ile Thr Lys Pro Leu Thr Tyr Asn Thr Leu Val Thr Pro Trp
   130 135 140
   Arg Leu Arg Leu Cys Ile Phe Leu Ile Trp Leu Tyr Ser Thr Leu Val
   145 150 155 160
   Phe Leu Pro Ser Phe Phe His Trp Gly Lys Pro Gly Tyr His Gly Asp
   165 170 175
   Val Phe Gln Trp Cys Ala Glu Ser Trp His Thr Asp Ser Tyr Phe Thr
   180 185 190
   Leu Phe Ile Val Met Met Leu Tyr Ala Pro Ala Ala Leu Ile Val Cys
   195 200 205
   Phe Thr Tyr Phe Asn Ile Phe Arg Ile Cys Gln Gln His Thr Lys Asp
   210 215 220
   Ile Ser Glu Arg Gln Ala Arg Phe Ser Ser Gln Ser Gly Glu Thr Gly
   225 230 235 240
   Glu Val Gln Ala Cys Pro Asp Lys Arg Tyr Ala Met Val Leu Phe Arg
   245 250 255
   Ile Thr Ser Val Phe Tyr Ile Leu Trp Leu Pro Tyr Ile Ile Tyr Phe
   260 265 270
   Leu Leu Glu Ser Ser Thr Gly His Ser Asn Arg Phe Ala Ser Phe Leu
   275 280 285
   Thr Thr Trp Leu Ala Ile Ser Asn Ser Phe Cys Asn Cys Val Ile Tyr
   290 295 300
   Ser Leu Ser Asn Ser Val Phe Gln Arg Gly Leu Lys Arg Leu Ser Gly
   305 310 315 320
   Ala Met Cys Thr Ser Cys Ala Ser Gln Thr Thr Ala Asn Asp Pro Tyr
   325 330 335
   Thr Val Arg Ser Lys Gly Pro Leu Asn Gly Cys His Ile
   340 345
(17) INFORMATION FOR SEQ ID NO:16:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:16:
      AGGAAGCTTT AAATTTCCAA GCCATGAATG 30
(18) INFORMATION FOR SEQ ID NO:17:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:17:
      ACCGAATTCA GATTACATTT GATTTACTAT G 31
(19) INFORMATION FOR SEQ ID NO:18:
   (i) SEQUENCE CHARACTERISTICS:
      (A) LENGTH: 1086 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:18:
   ATGAATGAAT CCAGGTGGAC TGAATGGAGG ATCCTGAACA TGAGCAGTGG CATTGTGAAT 60
   GTGTCCGAGC GTCACTCCTG CCCACTTGGA TTTGGCCACT ACAGTGTGGT GGATGTCTGC 120
   ATCTTCGAGA CAGTGGTTAT TGTGTTGCTG ACATTTCTGA TCATTGCTGG GAATCTAACA 180
   GTTATCTTTG TCTTTCATTG TGCTCCACTG TTACATCATT ATACTACCAG CTATTTCATT 240
   CAGACGATGG CATATGCTGA TCTTTTCGTT GGAGTTAGCT GCTTGGTTCC TACTCTGTCA 300
   CTTCTCCACT ACTCCACAGG TGTCCACGAG TCATTGACTT GCCAGGTTTT TGGATATATC 360
   ATCTCAGTTC TAAAAAGTGT TTCTATGGCA TGTCTTGCTT GCATCAGTGT GGATCGTTAT 420
   CTTGCAATAA CCAAGCCTCT TTCCTACAAT CAACTGGTCA CCCCTTGTCG CTTGAGAATT 480
   TGCATTATTT TGATCTGGAT CTACTCCTGC CTAATTTTCT TGCCTTCCTT TTTTGGCTGG 540
   GGGAAACCTG GTTACCATGG TGACATTTTT GAATGGTGTG CCACGTCTTG GCTCACCAGT 600
   GCCTATTTTA CTGGCTTTAT TGTTTGTTTA CTTTATGCTC CTGCTGCCTT TGTTGTCTGC 660
   TTCACTTACT TCCACATTTT CAAAATTTGC CGTCAGCACA CCAAAGAGAT AAATGACCGA 720
   AGAGCCCGAT TCCCTAGTCA TGAGGTAGAT TCTTCCAGAG AGACTGGACA CAGCCCTGAC 780
   CGTCGCTACG CCATGGTTTT GTTTAGGATA ACCAGTGTAT TTTATATGCT GTGGCTCCCC 840
   TATATAATTT ACTTTCTTCT AGAAAGCTCC CGGGTCTTGG ACAATCCAAC TCTGTCCTTC 900
   TTAACAACCT GGCTTGCAAT AAGTAATAGT TTTTGTAACT GTGTAATATA CAGCCTCTCC 960
   AACAGCGTTT TCCGGCTAGG CCTCCGAAGA CTGTCTGAGA CAATGTGCAC ATCCTGTATG 1020
   TGTGTGAAGG ATCAGGAAGC ACAAGAACCC AAACCTAGGA AACGGGCTAA TTCTTGCTCC 1080
   ATTTGA 1086
(20) INFORMATION FOR SEQ ID NO:19:
   (i) SEQUENCE CHARACTERISTICS:
      (A) LENGTH: 361 amino acids
      (B) TYPE: amino acid
      (C) STRANDEDNESS:
      (D) TOPOLOGY: not relevant
   (ii) MOLECULE TYPE: protein
   (xi) SEQUENCE DESCRIPTION: SEQ ID NO:19:
   Met Asn Glu Ser Arg Trp Thr Glu Trp Arg Ile Leu Asn Met Ser Ser
   1               5                   10                  15
   Gly Ile Val Asn Val Ser Glu Arg His Ser Cys Pro Leu Gly Phe Gly
               20                  25                  30
   His Tyr Ser Val Val Asp Val Cys Ile Phe Glu Thr Val Val Ile Val
           35                  40                  45
   Leu Leu Thr Phe Leu Ile Ile Ala Gly Asn Leu Thr Val Ile Phe Val
       50                  55                  60
   Phe His Cys Ala Pro Leu Leu His His Tyr Thr Thr Ser Tyr Phe Ile
   65 70 75 80
   Gln Thr Met Ala Tyr Ala Asp Leu Phe Val Gly Val Ser Cys Leu Val
   85 90 95
   Pro Thr Leu Ser Leu Leu His Tyr Ser Thr Gly Val His Glu Ser Leu
   100 105 110
   Thr Cys Gln Val Phe Gly Tyr Ile Ile Ser Val Leu Lys Ser Val Ser
   115 120 125
   Met Ala Cys Leu Ala Cys Ile Ser Val Asp Arg Tyr Leu Ala Ile Thr
   130 135 140
   Lys Pro Leu Ser Tyr Asn Gln Leu Val Thr Pro Cys Arg Leu Arg Ile
   145 150 155 160
   Cys Ile Ile Leu Ile Trp Ile Tyr Ser Cys Leu Ile Phe Leu Pro Ser
   165 170 175
   Phe Phe Gly Trp Gly Lys Pro Gly Tyr His Gly Asp Ile Phe Glu Trp
   180 185 190
   Cys Ala Thr Ser Trp Leu Thr Ser Ala Tyr Phe Thr Gly Phe Ile Val
   195 200 205
   Cys Leu Leu Tyr Ala Pro Ala Ala Phe Val Val Cys Phe Thr Tyr Phe
   210 215 220
   His Ile Phe Lys Ile Cys Arg Gln His Thr Lys Glu Ile Asn Asp Arg
   225 230 235 240
   Arg Ala Arg Phe Pro Ser His Glu Val Asp Ser Ser Arg Glu Thr Gly
   245 250 255
   His Ser Pro Asp Arg Arg Tyr Ala Met Val Leu Phe Arg Ile Thr Ser
   260 265 270
   Val Phe Tyr Met Leu Trp Leu Pro Tyr Ile Ile Tyr Phe Leu Leu Glu
   275 280 285
   Ser Ser Arg Val Leu Asp Asn Pro Thr Leu Ser Phe Leu Thr Thr Trp
   290 295 300
   Leu Ala Ile Ser Asn Ser Phe Cys Asn Cys Val Ile Tyr Ser Leu Ser
   305 310 315 320
   Asn Ser Val Phe Arg Leu Gly Leu Arg Arg Leu Ser Glu Thr Met Cys
   325 330 335
   Thr Ser Cys Met Cys Val Lys Asp Gln Glu Ala Gln Glu Pro Lys Pro
   340 345 350
   Arg Lys Arg Ala Asn Ser Cys Ser Ile
   355 360
(21) INFORMATION FOR SEQ ID NO:20:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:20:
      AGCGAATTCT GCCCACCCCA CGCCGAGGTG CT 32
(22) INFORMATION FOR SEQ ID NO:21:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:21:
      TGCGGATCCG CCAGCTCTTG AGCCTGCACA 30
(23) INFORMATION FOR SEQ ID NO:22:
   (i) SEQUENCE CHARACTERISTICS:
      (A) LENGTH: 1381 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:22:
(24) INFORMATION FOR SEQ ID NO:23:
   (i) SEQUENCE CHARACTERISTICS:
      (A) LENGTH: 407 amino acids
      (B) TYPE: amino acid
      (C) STRANDEDNESS:
      (D) TOPOLOGY: not relevant
   (ii) MOLECULE TYPE: protein (genomic)
   (xi) SEQUENCE DESCRIPTION: SEQ ID NO:23:
(25) INFORMATION FOR SEQ ID NO:24:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:24:
      GGAAGCTTCA GGCCCAAAGA TGGGGAACAT 30
(26) INFORMATION FOR SEQ ID NO:25:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:25:
      GTGGATCCAC CCGCGGAGGA CCCAGGCTAG 30
(27) INFORMATION FOR SEQ ID NO:26:
   (i) SEQUENCE CHARACTERISTICS:
      (A) LENGTH: 1697 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:26:
   ACTCCCAAAG TGCTGGGCTT ACAGGTGTAA GCCATCATGT CCAGCCGTTC AGATATTCTA 60
   GTTGAATTGG AGTTGGTGGG CTAGTACACC TTCTAAATTA AATGAGTAAA GGATTTAGAA 120
   TGGTGCCTGA CACACAGTAG GTGCTACATT CATGTTAGCT ACTATTATAA ACCTTTCCTG 180
   CCTCTGACTT TCAGGGTCTT GCCCACCACC AGCGATGCCC AGCCCTTGGT AGAGCTTGAA 240
   CCACCTTCTA TAAACAGGAT GGCGGTGGAG AGACAGGCCC AGTCCCTGAG CCCATGAGGA 300
   GTGTGGCCCC TTCAGGCCCA AAGATGGGGA ACATCACTGC AGACAACTCC TCGATGAGCT 360
   GTACCATCGA CCATACCATC CACCAGACGC TGGCCCCGGT GGTCTATGTT ACCGTGCTGG 420
   TGGTGGGCTT CCCGGCCAAC TGCCTGTCCC TCTACTTCGG CTACCTGCAG ATCAAGGCCC 480
   GGAACGAGCT GGGCGTGTAC CTGTGCAACC TGACGGTGGC CGACCTCTTC TACATCTGCT 540
   CGCTGCCCTT CTGGCTGCAG TACGTGCTGC AGCACGACAA CTGGTCTCAC GGCGACCTGT 600
   CCTGCCAGGT GTGCGGCATC CTCCTGTACG AGAACATCTA CATCAGCGTG GGCTTCCTCT 660
   GCTGCATCTC CGTGGACCGC TACCTGGCTG TGGCCCATCC CTTCCGCTTC CACCAGTTCC 720
   GGACCCTGAA GGCGGCCGTC GGCGTCAGCG TGGTCATCTG GGCCAAGGAG CTGCTGACCA 780
   GCATCTACTT CCTGATGCAC GAGGAGGTCA TCGAGGACGA GAACCAGCAC CGCGTGTGCT 840
   TTGAGCACTA CCCCATCCAG GCATGGCAGC GCGCCATCAA CTACTACCGC TTCCTGGTGG 900
   GCTTCCTCTT CCCCATCTGC CTGCTGCTGG CGTCCTACCA GGGCATCCTG CGCGCCGTGC 960
   GCCGGAGCCA CGGCACCCAG AAGAGCCGCA AGGACCAGAT CCAGCGGCTG GTGCTCAGCA 1020
   CCGTGGTCAT CTTCCTGGCC TGCTTCCTGC CCTACCACGT GTTGCTGCTG GTGCGCAGCG 1080
   TCTGGGAGGC CAGCTGCGAC TTCGCCAAGG GCGTTTTCAA CGCCTACCAC TTCTCCCTCC 1140
   TGCTCACCAG CTTCAACTGC GTCGCCGACC CCGTGCTCTA CTGCTTCGTC AGCGAGACCA 1200
   CCCACCGGGA CCTGGCCCGC CTCCGCGGGG CCTGCCTGGC CTTCCTCACC TGCTCCAGGA 1260
   CCGGCCGGGC CAGGGAGGCC TACCCGCTGG GTGCCCCCGA GGCCTCCGGG AAAAGCGGGG 1320
   CCCAGGGTGA GGAGCCCGAG CTGTTGACCA AGCTCCACCC GGCCTTCCAG ACCCCTAACT 1380
   CGCCAGGGTC GGGCGGGTTC CCCACGGGCA GGTTGGCCTA GCCTGGGTCC TCCGCGGGTG 1440
   GCTCCACGTG AGGCCTGAGC CTTCAGCCCA CGGGCCTCAG GGCCTGCCGC CTCCTGCTTC 1500
   CCTCGCTGCG GAGGCAGGGA AGCCCCTGTA ACTCCGGAAG CCTGCTCTCG CTTGCTGAGC 1560
   CCGCTGGGAC CGCCGAGGGT GGGAATAAGC CCCGGTTGGC TCGTGGGAAT AAGCCGTGTC 1620
   CTCTGCCGCG GCTGCGATGT GGCCACGCTG GGGCTGCTGG TCGGGGGAAA ACAGTGAACT 1680
   GCGTCCCCTG GCCTGCT 1697
(28) INFORMATION FOR SEQ ID NO:27:
   (i) SEQUENCE CHARACTERISTICS:
      (A) LENGTH: 365 amino acids
      (B) TYPE: amino acid
      (C) STRANDEDNESS:
      (D) TOPOLOGY: not relevant
   (ii) MOLECULE TYPE: protein (genomic)
   (xi) SEQUENCE DESCRIPTION: SEQ ID NO:27:
(29) INFORMATION FOR SEQ ID NO:28:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:28:
      CTGGTCCTGC ACTTTGCTGC 20
(30) INFORMATION FOR SEQ ID NO:29:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:29:
      AGCATCACAT AGGTCCGTGT CAC 23
(31) INFORMATION FOR SEQ ID NO:30:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:30:
      ACCAGAAAGG GTGTGGGTAC ACTG 24
(32) INFORMATION FOR SEQ ID NO:31:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:31:
      GGAACGAAAG GGCACTTTGG 20
(33) INFORMATION FOR SEQ ID NO:32:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:32:
      GCTGCCTCGG GATTATTTAG 20
(34) INFORMATION FOR SEQ ID NO:33:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:33:
      GCCTATTAGC AGGAACATGGGTG 23
(35) INFORMATION FOR SEQ ID NO:34:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:34:
      GCTAGCGTTC ATCGCCGC 18
(36) INFORMATION FOR SEQ ID NO:35:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:35:
      CTGGACTGTA TCGCCCCG 18
(37) INFORMATION FOR SEQ ID NO:36:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:36:
      GATCTCTAGA ATGATGTGGG GTGCAGGCAG CC 32
(38) INFORMATION FOR SEQ ID NO:37:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:37:
      CTAGGGTACC CGGACATCAC TGGGGGAGCG GGATC 35
(39) INFORMATION FOR SEQ ID NO:38:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:38:
      GATCTCTAGA ATGCAGGGTG CAAATCCGGC C 31
(40) INFORMATION FOR SEQ ID NO:39:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:39:
      CTAGGGTACC CGGACCTCGC TGGGAGACCT GGAAC 35
(41) INFORMATION FOR SEQ ID NO:40:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:40:
      ATGTGGAACG CGACGCCCAG CG 22
(42) INFORMATION FOR SEQ ID NO:41:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:41:
      TCATGTATTA ATACTAGATT CT 42
(43) INFORMATION FOR SEQ ID NO:42:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:42:
      TACCATGTGG AACGCGACGC CCAGCGAAGA GCCGGGGT 38
(44) INFORMATION FOR SEQ ID NO:43:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:43:
      CGGAATTCAT GTATTAATAC TAGATTCTGT CCAGGCCCG 39
(45) INFORMATION FOR SEQ ID NO:44:
   (i) SEQUENCE CHARACTERISTICS:
      (A) LENGTH: 1101 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:44:
   ATGTGGAACG CGACGCCCAG CGAAGAGCCG GGGTTCAACC TCACACTGGC CGACCTGGAC 60
   TGGGATGCTT CCCCCGGCAA CGACTCGCTG GGCGACGAGC TGCTGCAGCT CTTCCCCGCG 120
   CCGCTGCTGG CGGGCGTCAC AGCCACCTGC GTGGCACTCT TCGTGGTGGG TATCGCTGGC 180
   AACCTGCTCA CCATGCTGGT GGTGTCGCGC TTCCGCGAGC TGCGCACCAC CACCAACCTC 240
   TACCTGTCCA GCATGGCCTT CTCCGATCTG CTCATCTTCC TCTGCATGCC CCTGGACCTC 300
   GTTCGCCTCT GGCAGTACCG GCCCTGGAAC TTCGGCGACC TCCTCTGCAA ACTCTTCCAA 360
   TTCGTCAGTG AGAGCTGCAC CTACGCCACG GTGCTCACCA TCACAGCGCT GAGCGTCGAG 420
   CGCTACTTCG CCATCTGCTT CCCACTCCGG GCCAAGGTGG TGGTCACCAA GGGGCGGGTG 480
   AAGCTGGTCA TCTTCGTCAT CTGGGCCGTG GCCTTCTGCA GCGCCGGGCC CATCTTCGTG 540
   CTAGTCGGGG TGGAGCACGA GAACGGCACC GACCCTTGGG ACACCAACGA GTGCCGCCCC 600
   ACCGAGTTTG CGGTGCGCTC TGGACTGCTC ACGGTCATGG TGTGGGTGTC CAGCATCTTC 660
   TTCTTCCTTC CTGTCTTCTG TCTCACGGTC CTCTACAGTC TCATCGGCAG GAAGCTGTGG 720
   CGGAGGAGGC GCGGCGATGC TGTCGTGGGT GCCTCGCTCA GGGACCAGAA CCACAAGCAA 780
   ACCGTGAAAA TGCTGGCTGT AGTGGTGTTT GCCTTCATCC TCTGCTGGCT CCCCTTCCAC 840
   GTAGGGCGAT ATTTATTTTC CAAATCCTTT GAGCCTGGCT CCTTGGAGAT TGCTCAGATC 900
   AGCCAGTACT GCAACCTCGT GTCCTTTGTC CTCTTCTACC TCAGTGCTGC CATCAACCCC 960
   ATTCTGTACA ACATCATGTC CAAGAAGTAC CGGGTGGCAG TGTTCAGACT TCTGGGATTC 1020
   GAACCCTTCT CCCAGAGAAA GCTCTCCACT CTGAAAGATG AAAGTTCTCG GGCCTGGACA 1080
   GAATCTAGTA TTAATACATG A 1101
(46) INFORMATION FOR SEQ ID NO:45:
   (i) SEQUENCE CHARACTERISTICS:
      (A) LENGTH: 366 amino acids
      (B) TYPE: amino acid
      (C) STRANDEDNESS:
      (D) TOPOLOGY: not relevant
   (ii) MOLECULE TYPE: protein (genomic)
   (xi) SEQUENCE DESCRIPTION: SEQ ID NO:45:

## Description

### FIELD OF THE INVENTION

The invention disclosed in this patent document relates to transmembrane receptors, more particularly to endogenous, constitutively active G protein-coupled receptors for which the endogenous ligand is unknown, and most particularly to the use of such receptors for the direct identification of candidate compounds via screening as agonists, partial agonists or inverse agonists to such receptors.

### BACKGROUND OF THE INVENTION

### A. G protein-coupled receptors

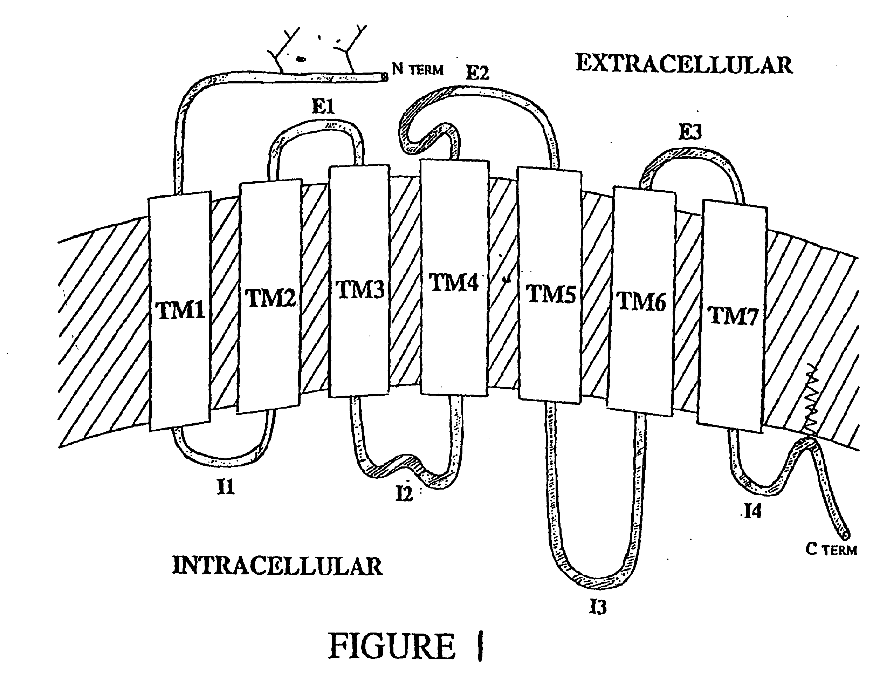

G protein-coupled receptors share a common structural motif. All these receptors have seven sequences of between 22 to 24 hydrophobic amino acids that form seven alpha helices, each of which spans the membrane. The transmembrane helices are joined by strands of amino acids having a larger loop between the fourth and fifth transmembrane helix on the extracellular side of the membrane. Another larger loop, composed primarily of hydrophilic amino acids, joins transmembrane helices five and six on the intracellular side of the membrane. The carboxy terminus of the receptor lies intracellularly with the amino terminus in the extracellular space. It is thought that the loop joining helices five and six, as well as the carboxy terminus, interact with the G protein. Currently, Gq, Gs, Gi, and Go are G proteins that have been identified. The general structure of G protein-coupled receptors is shown in Figure 1.

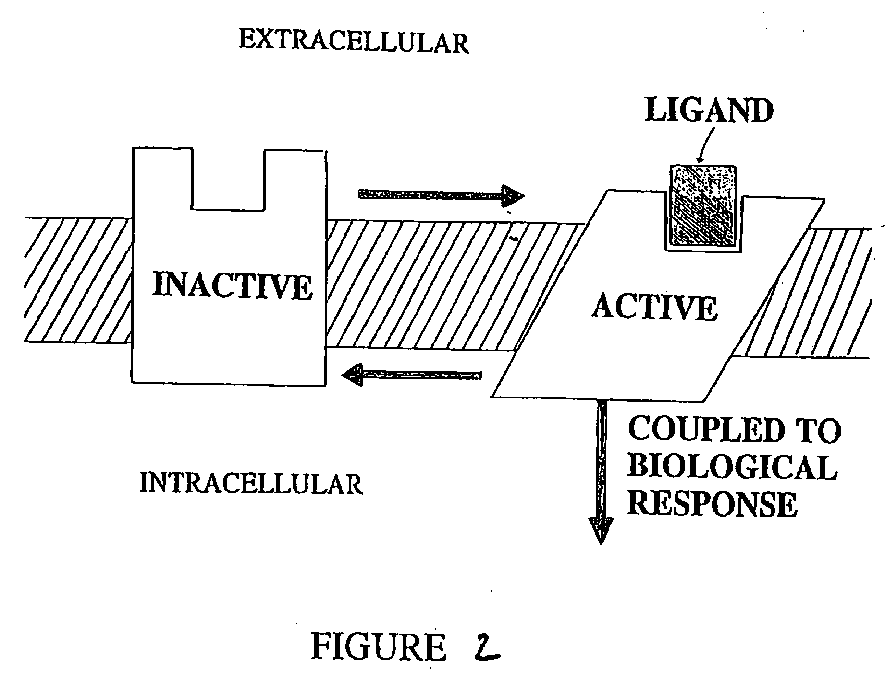

Under physiological conditions, G protein-coupled receptors exist in the cell membrane in equilibrium between two different states or conformations: an "inactive" state and an "active" state. As shown schematically in Figure 2, a receptor in an inactive state is unable to link to the intracellular transduction pathway to produce a biological response. Changing the receptor conformation to the active state allows linkage to the transduction pathway and produces a biological response.

A receptor may be stabilized in an active state by an endogenous ligand or an exogenous agonist ligand. Recent discoveries such as, including but not exclusively limited to, modifications to the amino acid sequence of the receptor provide means other than ligands to stabilize the active state conformation. These means effectively stabilize the receptor in an active state by simulating the effect of a ligand binding to the receptor. Stabilization by such ligand-independent means is termed "constitutive receptor activation." A receptor for which the endogenous ligand is unknown or not identified is referred to as an "orphan receptor."

### B. Traditional Compound Screening

Generally, the use of an orphan receptor for screening purposes to identify compounds that modulate a biological response associated with such receptor has not been possible. This is because the traditional "dogma" regarding screening of compounds mandates that the ligand for the receptor be known, whereby compounds that competitively bind with the receptor, *i*.*e*., by interfering or blocking the binding of the natural ligand with the receptor, are selected. By definition, then, this approach has no applicability with respect to orphan receptors. Thus, by adhering to this dogmatic approach to the discovery of therapeutics, the art, in essence, has taught and has been taught to forsake the use of orphan receptors unless and until the natural ligand for the receptor is discovered. The pursuit of an endogenous ligand for an orphan receptor can take several years and cost millions of dollars.

Furthermore, and given that there are an estimated 2,000 G protein-coupled receptors in the human genome, the majority of which being orphan receptors, the traditional dogma castigates a creative approach to the discovery of therapeutics to these receptors.

### C. Exemplary Orphan Receptors: GPR3, GPR4, GPR6, GPR12, GPR21, GHSR, OGR1 and AL022171

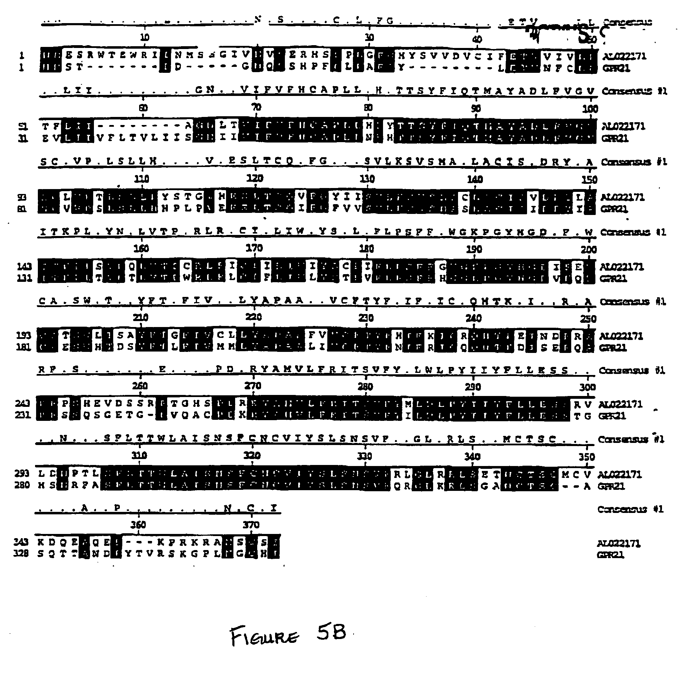

GPR3 is a 330 amino acid G protein coupled receptor for which the endogenous ligand is unknown. (Marchese, A. et al. (1994) Genomics 23:609; *see also*, Iismaa, T.P. et al (1994) Genomics 24:391; *see* Figure 1 for reported nucleic acid and amino acid sequence.) GPR3 is constitutively active in its endogenous form. (Eggerick, D. et al. (1995) Biochem. J. 389:837). GPR12 is a 334 amino acid homolog of GPR3; the endogenous ligand for GPR12 is unknown (Song, Z. -H., et al (1995) Genomics, 28:347; *see* Figure 1 for reported amino acid sequence). GPR6 is a 362 amino acid homolog of GPR3; the endogenous ligand for GPR6 is unknown (Song, Z.-H. et al, *supra*.; *see* Figure 1 for reported amino acid sequence). GPR6 transcripts are reported to be abundant in the human putamen and to a lesser extent in the frontal cortex, hippocampus, and hypothalamus (Heiber, M. et al. DNA and Cell Biology (1995) 14(1): 25; *see* Figure 1 for reported nucleic acid and amino acid sequences for GPR6). GPR4 has also been identified as an orphan GPCR (Heiber, M. et al, 14 DNA Cell Biol. 25 (1995)). OGR1, an orphan GPCR, is reported to have a high level of homology with GPR4 (Xu, Y. and Casey, G., 35 Genomics 397 (1996)). GPR21 is a 349 amino acid G protein coupled receptor for which the endogenous ligand is unknown (*see* GenBank Accession # U66580 for nucleic acid and deduced amino acid sequence). GPR21 has been reported to be located at chromosome 9q33. O'Dowd B. et al., 187 Gene 75 (1997). AL022171 is a human DNA sequence from clone 384F21 on chromosome 1q24. AL022171 has been identified to contain an open reading frame of 1,086 bp encoding for a 361 amino acid protein. (*see* GenBank Accession number AL022171). AL022171 is 68% homologous to GPR21 (*see* Figure 5B). GHSR is also identified as an orphan GPCR (Howard, A.D. et al, 273 Science 974 (1996)).

WO97/21731 discloses a method for identifying agonists, partial agonists, inverse agonists or antagonists of a peptide hormone receptor (a G-protein coupled receptor), comprising measuring the binding affinity of a candidate compound to a constitutively active form of the receptor.

### SUMMARY OF THE INVENTION

Disclosed herein are methods for screening of candidate compounds against endogenous, constitutively activated G protein-coupled orphan receptors (GPCRs) for the direct identification of candidate compounds as agonists, inverse agonists or partial agonists to such receptors. For such screening purposes, an endogenous, constitutively activated orphan GPCR:G protein - fusion protein is utilized.

### BRIEF DESCRIPTION OF THE DRAWINGS

**Figure 1** shows a generalized structure of a G protein-coupled receptor with the numbers assigned to the transmembrane helixes, the intracellular loops, and the extracellular loops.
**Figure 2** schematically shows the two states, active and inactive, for a typical G protein coupled receptor and the linkage of the active state to the second messenger transduction pathway.
**Figure 3** is computerized representation of a "dot-blot" showing the distribution of the orphan receptor GPR4 across a variety of human tissues (*see* Appendix A for grid-code).
**Figure 4** is a diagram showing enhanced binding of [³⁵S]GTγS to membranes prepared from 293T cells transfected with the orphan receptor GPR3 compared to those transfected with control vector alone at 75 µg/well membrane protein. The radiolabeled concentration of [³⁵S]GTPγS was held constant at 1.2 nM and the GDP concentration was held constant at 1 µM. The assay was performed on 96-well format in Wallac scintistrips.
**Figure 5A** shows the amino acid alignment of orphan receptors GPR3, GPR6, and GPR12. **Figure 5B** shows the amino acid alignment of orphan receptors GPR21 and A1022171 (Consensus #1 indicates matching residues).
**Figure 6A** is a diagram showing that the orphan receptors GPR3, GPR6, and GPR 12 are confirmed to be constitutively active by their enhanced ability to induce expression of β-galactosidase from a CRE driven reporter system in VIP cells. Figure 6B and 6C are diagrams of orphan receptors GPR21 and AL022171, respectively, that have also been confirmed to be constitutively active by their enhanced ability to induce expression of the luciferase gene from a CRE driven reporter system in both 293 and 293T cells.
**Figures 7A, 7B** and **7C** show the relative distribution of the expression of the GPR3 (A), GPR6 (B), and GPR12 (C) orphan receptors across several normal human tissues as determined by RT-PCR. Abbreviations: Ocx = occipital cortex; Hypoth = hypothalamus; Tex = temporal cortex; Fcx = frontal cortex.
**Figures 8A** and **8B** show GPR3 receptor expression in normal (A) and epileptic (B) human brain tissue as examined by RT-PCR.
**Figure 9A** is a copy of an autoradiograph evidencing the results from in situ hybridization (normal rat) using GPR6 probe; **Figure 9B** is a reference map of the corresponding region of the rat brain.
**Figure 10A** is a copy of an autoradiograph evidencing the results from in situ hybridization (Zucker rat - lean) using GPR6 probe; **Figure 10B** is a copy of an autoradiograph evidencing the results from in situ hybridization (Zucker rat - obese) using GPR6 probe; **Figure 10C** is a reference map of the corresponding region of the rat brain.
**Figures 11A-F** are copies of autoradiographs evidencing the results from in situ hybridization (normal rat) using GPR12 probe.
**Figure 12** is a copy of an autoradiograph evidencing the results from in situ hybridization (normal rat) using GPR6 probe (12A), and orexin 1 receptor probe (12B) with overlays for determination of co-localization of the two receptors (12C and 12D).
**Figure 13** is a copy of an autoradiograph evidencing the results from in situ hybridization (normal rat) using GPR6 probe (13A), and melanocortin-3 receptor probe (13B) with overlays for determination of co-localization of the two receptors (13C and 13D).
**Figure 14** provides results from co-localization experiment, evidencing that GPR6 and AGRP are co-localized within the arcuate. The arrow directs attention to to a specific cell within the arcuate, with the circle surrounding the cell; the "dots" are radiolabeled GPR6, and beneath those, in a darker shade, is AGRP.
**Figure 15** provides graphic results of body weight over time from animals (n = 5) receiving antisense oligonucleotides to GPR6 (star symbol at Day 5 indicates day on which animals received d-amphetamine sulfate injection; *see* **Figure 16**).
**Figure 16** provides bar graph results from baseline locomotor activity and from amphetamine-induced locomotive behavior in the animals of **Figure 15****.**
**Figure 17** provides bar-graph results from the direct identification of candidate compounds screened against GPR3 Fusion Protein **(****Figure 17A****)** and GPR6 Fusion Protein **(****Figure 17B****).**
**Figure 18A-L** is a sequence diagram of the preferred vector pCMV, including restriction enzyme site locations.

### DETAILED DESCRIPTION

The scientific literature that has evolved around receptors has adopted a number of terms to refer to ligands having various effects on receptors. For clarity and consistency, the following definitions will be used throughout this patent document. To the extent that these definitions conflict with other definitions for these terms, the following definitions shall control:
**AGONISTS** shall mean materials (*e*.*g*., ligands, candidate compounds) that activate the intracellular response when they bind to the receptor, or enhance GTP binding to membranes.
**AMINO ACID ABBREVIATIONS** used herein are set out in Table 1:

| **TABLE 1** | | |
|---|---|---|
| ALANINE | ALA | A |
| ARGININE | ARG | R |
| ASPARAGINE | ASN | N |
| ASPARTIC ACID | ASP | D |
| CYSTEINE | CYS | C |
| GLUTAMIC ACID | GLU | E |
| GLUTAMINE | GLN | Q |
| GLYCINE | GLY | G |
| HISTIDINE | HIS | H |
| ISOLEUCINE | ILE | I |
| LEUCINE | LEU | L |
| LYSINE | LYS | K |
| METHIONINE | MET | M |
| PHENYLALANINE | PHE | F |
| PROLINE | PRO | P |
| SERINE | SER | S |
| THREONINE | THR | T |
| TRYPTOPHAN | TRP | W |
| TYROSINE | TYR | Y |
| VALINE | VAL | V |

**PARTIAL AGONISTS** shall mean materials (*e*.*g*., ligands, candidate compounds) which activate the intracellular response when they bind to the receptor to a lesser degree/extent than do agonists, or enhance GTP binding to membranes to a lesser degree/extent than do agonists
**ANTAGONIST** shall mean materials (*e*.*g*., ligands, candidate compounds) that competitively bind to the receptor at the same site as the agonists but which do not activate the intracellular response initiated by the active form of the receptor, and can thereby inhibit the intracellular responses by agonists or partial agonists. ANTAGONISTS do not diminish the baseline intracellular response in the absence of an agonist or partial agonist.
**CANDIDATE COMPOUND** shall mean a molecule (for example, and not limitation, a chemical compound) which is amenable to a screening technique. Preferably, the phrase "candidate compound" does not include compounds which were publicly known to be compounds selected from the group consisting of inverse agonist, agonist or antagonist to a receptor, as previously determined by an indirect identification process ("indirectly identified compound"); more preferably, not including an indirectly identified compound which has previously been determined to have therapeutic efficacy in at least one mammal; and, most preferably, not including an indirectly identified compound which has previously been determined to have therapeutic utility in humans.
**COMPOSITION** means a material comprising at least one component; a "pharmaceutical composition" is an example of a composition.
**COMPOUND EFFICACY** shall mean a measurement of the ability of a compound to inhibit or stimulate receptor functionality, as opposed to receptor binding affinity. A most preferred means of detecting compound efficacy is via measurement of GTP (via [³⁵S]GTPγS) or cAMP, as further disclosed in the Example section of this patent document.
**CONSTITUTIVELY ACTIVATED RECEPTOR** (Constitutively Active Receptor) shall mean a receptor subject to constitutive receptor activation. A constitutively activated receptor can be endogenous or non-endogenous.
**CONSTITUTIVE RECEPTOR ACTIVATION** shall mean stabilization of a receptor in the active state by means other than binding of the receptor with its endogenous ligand or a chemical equivalent thereof.
**CONTACT** or **CONTACTING** shall mean bringing at least two moieties together, whether in an in vitro system or an in vivo system.
**DIRECTLY IDENTIFYING or DIRECTLY IDENTIFIED,** in relationship to the phrase "candidate compound", shall mean the screening of a candidate compound against a constitutively activated receptor, preferably a constitutively activated orphan receptor, and most preferably against a constitutively activated G protein-coupled cell surface orphan receptor, and assessing the compound efficacy of such compound. This phrase is, under no circumstances, to be interpreted or understood to be encompassed by or to encompass the phrase "indirectly identifying" or "indirectly identified."
**ENDOGENOUS** shall mean a material that a mammal naturally produces. ENDOGENOUS in reference to, for example and not limitation, the term "receptor," shall mean that which is naturally produced by a mammal (for example, and not limitation, a human) or a virus. By contrast, the term **NON-ENDOGENOUS** in this context shall mean that which is not naturally produced by a mammal (for example, and not limitation, a human) or a virus. For example, and not limitation, a receptor which is not constitutively active in its endogenous form, but when manipulated becomes constitutively active, is most preferably referred to herein as a "non-endogenous, constitutively activated receptor." Both terms can be utilized to describe both "in vivo" and "in vitro" systems. For example, and not limitation, in a screening approach, the endogenous or non-endogenous receptor may be in reference to an in vitro screening system. As a further example and not limitation, where the genome of a mammal has been manipulated to include a non-endogenous constitutively activated receptor, screening of a candidate compound by means of an in vivo system is viable.
**G PROTEIN COUPLED RECEPTOR FUSION PROTEIN** and **GPCR FUSION PROTEIN,** in the context of the invention disclosed herein, each mean a non-endogenous protein comprising an endogenous, constitutively activated orphan GPCR fused to at least one G protein, most preferably, the alpha (α) subunit of such G protein (this being the subunit that binds GTP), with the G protein preferably being of the same type as the G protein that naturally couples with endogenous orphan GPCR For example, and not limitation, in an endogenous state, the G protein "Gsα" is the predominate G protein that couples with GPR6 such that a GPCR Fusion Protein based upon GPR6 would be a non-endogenous protein comprising GPR6 fused to Gsα. The G protein can be fused directly to the c-terminus of the endogenous, constitutively active orphan GPCR, or there may be spacers between the two.
**INDIRECTLY IDENTIFYING or INDIRECTLY IDENTIFIED** means the traditional approach to the drug discovery process involving identification of an endogenous ligand specific for an endogenous receptor, screening of candidate compounds against the receptor for determination of those which interfere and/or compete with the ligand-receptor interaction, and assessing the efficacy of the compound for affecting at least one second messenger pathway associated with the activated receptor.
**INHIBIT** or **INHIBITING**, in relationship to the term "response" shall mean that a response is decreased or prevented in the presence of a compound as opposed to in the absence of the compound.
**INVERSE AGONISTS** shall mean materials (*e*.*g*., ligand, candidate compound) which bind to either the endogenous form of the receptor or to the constitutively activated form of the receptor, and which inhibit the baseline intracellular response initiated by the active form of the receptor below the normal base level of activity which is observed in the absence of agonists or partial agonists, or decrease GTP binding to membranes. Preferably, the baseline intracellular response is inhibited in the presence of the inverse agonist by at least 30%, more preferably by at least 50%, and most preferably by at least 75%, as compared with the baseline response in the absence of the inverse agonist.
**LIGAND** shall mean an endogenous, naturally occurring molecule specific for an endogenous, naturally occurring receptor.
**ORPHAN RECEPTOR** shall mean an endogenous receptor for which the endogenous ligand specific for that receptor has not been identified or is not known.
**PHARMACEUTICAL COMPOSITION** shall mean a composition comprising at least one active ingredient, whereby the composition is amenable to investigation for a specified, efficacious outcome in a mammal (for example, and not limitation, a human). Those of ordinary skill in the art will understand and appreciate the techniques appropriate for determining whether an active ingredient has a desired efficacious outcome is based upon the needs of the artisan.
**NON-ORPHAN RECEPTOR** shall mean an endogenous naturally occurring molecule specific for an endogenous naturally occurring ligand wherein the binding of a ligand to a receptor activates an intracellular signaling pathway.
**STIMULATE** or **STIMULATING,** in relationship to the term "response" shall mean that a response is increased in the presence of a compound as opposed to in the absence of the compound.
The order of the following sections is set forth for presentational efficiency and is not intended, nor should be construed, as a limitation on the disclosure or the claims to follow.

### A. Introduction

The traditional study of receptors has always proceeded from the a priori assumption (historically based) that the endogenous ligand must first be identified before discovery could proceed to find antagonists and other molecules that could affect the receptor. Even in cases where an antagonist might have been known first, the search immediately extended to looking for the endogenous ligand. This mode of thinking has persisted in receptor research even after the discovery of constitutively activated receptors. What has not been heretofore recognized is that it is the active state of the receptor that is most useful for discovering agonists, partial agonists, and inverse agonists of the receptor. For those diseases which result from an overly active receptor, what is desired in a therapeutic drug is a compound which acts to diminish the active state of a receptor, not necessarily a drug which is an antagonist to the endogenous ligand. This is because a compound (drug) which reduces the activity of the active receptor state need not bind at the same site as the endogenous ligand. Thus, as taught by a method of this invention, any search for therapeutic compounds should start by screening compounds against the ligand-independent active state. The search, then, is for an inverse agonist to the active state receptor.

Screening candidate compounds against the endogenous, constitutively activated orphan receptors, for example, and not limited to, the endogenous, constitutively active GPCRs set forth herein, GPR3, GPR4, GPR6, GPR12, GPR21, GHSR, OGR1, RE2 and AL022171, allows for the direct identification of candidate compounds which act at these orphan cell surface receptors, without requiring any prior knowledge or use of the receptor's endogenous ligand. By determining areas within the body where such receptors are expressed and/or over-expressed, it is possible to determine related disease/disorder states which are associated with the expression and/or over-expression of these receptors; such an approach is disclosed in this patent document.

### B. Disease/Disorder Identification and/or Selection

As will be set forth in greater detail below, most preferably inverse agonists to endogenous, constitutively activated orphan receptors, *e*.*g*., such as those set forth herein (GPR3, GPR4, GPR6, GPR12, GPR21, GHSR, OGR1, RE2 and AL022171) can be identified by the methodologies of this invention. Such inverse agonists are ideal candidates as lead compounds in drug discovery programs for treating diseases related to these receptors. Indeed, an antagonist to such a receptor (even if the ligand were known) may be ineffective given that the receptor is activated even in the absence of ligand-receptor binding. Because of the ability to directly identify inverse agonists to these receptors, thereby allowing for the development of pharmaceutical compositions, a search, for diseases and disorders associated with these receptors is possible. For example, scanning both diseased and normal tissue samples for the presence of these orphan receptors now becomes more than an academic exercise or one which might be pursued along the path of identifying an endogenous ligand. Tissue scans can be conducted across a broad range of healthy and diseased tissues. Such tissue scans provide a preferred first step in associating a specific receptor with a disease and/or a disorder.

Preferably, the DNA sequence of the endogenous, constitutively activated GPCR is used to make a probe for RT-PCR identification of the expression of the receptor in tissue samples. The presence of a receptor in a diseased tissue, or the presence of the receptor at elevated concentrations in diseased tissue compared to normal tissue, can be utilized to identify a correlation with that disease. Receptors can equally well be localized to regions of organs by this technique. Based on the known functions of the specific tissues to which the receptor is localized, the putative functional role of the receptor can be deduced.

### C. Homology Identification

The identification and association of an orphan receptor with diseases and/or disorders can be beneficially enhanced via identification of additional receptors having homology with the original orphan receptor. This approach was utilized in the identification of both GPR6 and GPR12, based upon their sequence homology with GPR3, and in the identification of AL022171, having sequence homology to GPR21. GPR3 was previously identified as a constitutively activated orphan receptor (*see* Eggerick, *supra*). What was not known, prior to this invention, was that GPR6, GPR12, GPR21 and AL022171 are also constitutively active in their endogenous states. Using known computerized databases (*e*.*g*., dbEST), GPR6, GPR12, GPR21 and AL022171 were identified.

This highlights certain unique benefits of the invention disclosed herein: because the dogma in drug screening relies upon knowledge and identification of a receptor's endogenous ligand, the art had no motivation to explore whether or not GPR3 homologs were constitutively active in their endogenous forms (other than for, at best, academic curiosity). However, with the power of the present invention to directly identify inverse agonists to such receptors, coupled with the ability to locate the distribution of such receptors in tissue samples, the present invention dramatically transcends such idle curiosity and provides a means for alleviating diseases and disorders which impact the human condition.

### D. Screening of Candidate Compounds

### 1. Generic GPCR screening assay techniques

When a G protein receptor becomes constitutively active, it binds to a G protein (eg., Gq, Gs, Gi, Go) and stimulates the binding of GTP to the G protein. The G protein then acts as a GTPase and slowly hydrolyzes the GTP to GDP, whereby the receptor, under normal conditions, becomes deactivated. However, constitutively activated receptors continue to exchange GDP to GTP. A non-hydrolyzable analog of GTP, [³⁵S]GTPγS, can be used to monitor enhanced binding to membranes which express constitutively activated receptors. It is reported that [³⁵S]GTPγS can be used to monitor G protein coupling to membranes in the absence and presence of ligand. An example of this monitoring, among other examples well-known and available to those in the art, was reported by Traynor and Nahorski in 1995. The preferred use of this assay system is for initial screening of candidate compounds because the system is generically applicable to all G protein-coupled receptors regardless of the particular G protein that interacts with the intracellular domain of the receptor. It is in the context of the use of a GTP assay system that a GPCR Fusion Protein is preferably utilized.

### B 2. Specific GPCR screening assay techniques

Once candidate compounds are identified using the "generic" G protein-coupled receptor assay (i.e. an assay to select compounds that are agonists, partial agonists, or inverse agonists), further screening to confirm that the compounds have interacted at the receptor site is preferred. For example, a compound identified by the "generic" assay may not bind to the receptor, but may instead merely "uncouple" the G protein from the intracellular domain. In the case of GPR3, GPR4, GPR6, GPR12, GPR21, GHSR, OGR1, RE2 and AL022171, it has been determined that these receptors couple the G protein Gs. Gs stimulates the enzyme adenylyl cyclase (Gi, on the other hand, inhibits this enzyme). Adenylyl cyclase catalyzes the conversion of ATP to cAMP; thus, because these receptors are activated in their endogenous forms, increased levels of cAMP are associated therewith (on the other hand, endogenously activated receptors which couple the Gi protein are associated with decreased levels of cAMP). *See, generally,* "Indirect Mechanisms of Synaptic Transmission," Chpt. 8, From Neuron To Brain (3rd Ed.) Nichols, J.G. et al eds. Sinauer Associates, Inc. (1992). Thus, assays that detect cAMP can be utilized to determine if a candidate compound is an inverse agonist to the receptor (*i*.*e*., such a compound which contacts the receptor would decrease the levels of cAMP relative to the uncontacted receptor). A variety of approaches known in the art for measuring cAMP can be utilized; a most preferred approach relies upon the use of anti-cAMP antibodies. Another type of assay that can be utilized is a whole cell second messenger reporter system assay. Promoters on genes drive the expression of the proteins that a particular gene encodes. Cyclic AMP drives gene expression by promoting the binding of a cAMP-responsive DNA binding protein or transcription factor (CREB) which then binds to the promoter at specific sites called cAMP response elements and drives the expression of the gene. Reporter systems can be constructed which have a promoter containing multiple cAMP response elements before the reporter gene, e.g., β-galactosidase or luciferase. Thus, an activated Gs receptor such as GPR3 causes the accumulation of cAMP which then activates the gene and expression of the reporter protein. The reporter protein such as β-galactosidase or luciferase can then be detected using standard biochemical assays (see, for example, Chen et al. 1995). A cAMP assay is particularly preferred.

The foregoing specific assay approach can, of course, be utilized to initially directly identify candidate compounds, rather than by using the generic assay approach. Such a selection is primarily a matter of choice of the artisan. With respect to GPR6, use of a modified, commercially available cAMP assay was initially utilized for the direct identification of inverse agonists.

### C 3. GPCR Fusion Protein

The use of an endogenous, constitutively activated orphan GPCR for use in screening of candidate compounds for the direct identification of inverse agonists, agonists and partial agonists provides a unique challenge in that, by definition, the endogenous receptor is active even in the absence of an endogenous ligand bound thereto. Thus, in order to differentiate between, *e*.*g*., the endogenous receptor in the presence of a candidate compound and the endogenous receptor in the absence of that compound, with an aim of such a differentiation to allow for an understanding as to whether such compound may be an inverse agonist, agonist, partial agonist or have no affect on such a receptor, it is preferred that an approach be utilized that can enhance such differentiation. A preferred approach is the use of a GPCR Fusion Protein.

Generally, once it is determined that an endogenous orphan GPCR is constitutively active, using the assay techniques set forth above (as well as others), it is possible to determine the predominant G protein that couples with the endogenous GPCR. Coupling of the G protein to the GPCR provides a signaling pathway that can be assessed. Because it is most preferred that screening take place by use of a mammalian expression system, such a system will be expected to have endogenous G protein therein. Thus, by definition, in such a system, the endogenous, constitutively active orphan GPCR will continuously signal. In this regard, it is preferred that this signal be enhanced such that in the presence of, *e*.*g*., an inverse agonist to the receptor, it is more likely that one will be able to more readily differentiate, particularly in the context of screening, between the receptor when it is or is not contacted with the inverse agonist.

The GPCR Fusion Protein is intended to enhance the efficacy of G protein coupling with the endogenous GPCR. The GPCR Fusion Protein appears to be important for screening with an endogenous, constitutively activated GPCR because such an approach increases the signal that is most preferably utilized in such screening techniques. Facilitating a significant "signal to noise" ratio is important for the screening of candidate compounds as disclosed herein.

The construction of a construct useful for expression of a GPCR Fusion Protein is within the purview of those having ordinary skill in the art. Commercially available expression vectors and systems offer a variety of approaches that can fit the particular needs of an investigator. One important criterion for such a GPCR Fusion Protein construct is that the endogenous GPCR sequence and the G protein sequence both be in-frame (preferably, the sequence for the endogenous GPCR is upstream of the G protein sequence) and that the "stop" codon of the GPCR must be deleted or replaced such that upon expression of the GPCR, the G protein can also be expressed. The GPCR can be linked directly to the G protein, or there can be spacer residues between the two (preferably no more than about 12, although this number can be readily ascertained by one of ordinary skill in the art). We have evaluated both approaches, and in terms of measurement of the activity of the GPCR, the results are substantially the same; however, there is a preference (based upon convenience) of use of a spacer in that some restriction sites that are not used will, effectively, upon expression, become a spacer. Most preferably, the G protein that couples to the endogenous GPCR will have been identified prior to the creation of the GPCR Fusion Protein construct. Because there are only a few G proteins that have been identified, it is preferred that a construct comprising the sequence of the G protein (*i*.*e*., a universal G protein construct) be available for insertion of an endogenous GPCR sequence therein; this provides for efficiency in the context of large-scale screening of a variety of different endogenous GPCRs having different sequences.

### E. Medicinal Chemistry

Generally, but not always, direct identification of candidate compounds is preferably conducted in conjunction with compounds generated via combinatorial chemistry techniques, whereby thousands of compounds are randomly prepared for such analysis. Generally, the results of such screening will be compounds having unique core structures; thereafter, these compounds are preferably subjected to additional chemical modification around a preferred core structure(s) to further enhance the medicinal properties thereof. In this way, inverse agonists, agonists and/or partial agonists that are directly identified can be beneficially improved upon prior to development of pharmaceutical compositions comprising such compounds. Generally, it is preferred that the binding affinity of a directly identified compound selected for further refinement into a pharmaceutical composition have a binding affinity for the receptor of less than 100nM, although this is generally a preference selection based upon the particular needs of the artisan. Such techniques are known to those in the art and will not be addressed in detail in this patent document.

### F. Pharmaceutical Compositions

Candidate compounds selected for further development can be formulated into pharmaceutical compositions using techniques well known to those in the art. Suitable pharmaceutically-acceptable carriers are available to those in the art; for example, see Remington's Pharmaceutical Sciences, 16th Edition, 1980, Mack Publishing Co., (Oslo et al., eds.).

### EXAMPLES

The following examples are presented for purposes of elucidation, and not limitation, of the present invention. While specific nucleic acid and amino acid sequences are disclosed herein, those of ordinary skill in the art are credited with the ability to make minor modifications to these sequences while achieving the same or substantially similar results reported below. It is intended that equivalent, endogenous, constitutively activated human orphan receptor sequences having eighty-five percent (85%) homology, more preferably having ninety percent (90%) homology, and most preferably having grater than ninety-five percent (95%) homology to GPR3, GPR4, GPR6, GPR12, GPR21, GHSR, OGR1, RE2 and AL022171 fall within the scope of any claims appended hereto.

### Example 1

### Preparation of In Situ Probes

In situ probes for GPR3, GPR6, and GPR12 were prepared. The following PCR protocol was utilized for all three probes: the reaction condition utilized was 1X rTth DNA polymerase buffer II, 1.5 mM Mg(OAc)₂, 0.2 mM each of the 4 nucleotides, 0.228 µg rat genomic DNA, 0.25 µM of each primer (see below) and 1 unit of rTth DNA polymerase (Perkin Elmer) in 50 µl reaction volume. The cycle condition was 30 cycles of 94°C for 1 min, 55 °C for 1 min and 72 °C for 45 sec with a Perkin Elmer Cetus 2400 thermal cycler.

### 1. Rat GPR3 in situ probe

Because the full length cDNA sequence for rat GPR3 is not data-base available, the DNA fragment for the in situ probe was obtained by PCR using a 3' degenerate oligonucleotide based on the published human and mouse GPR3 sequences in the middle of the transmembrane domain 3, and a 5' degenerate oligonucleotide near the beginning of the 5' extracellular domain. The sequences of the oligonucleotides utilized were as follows: 5'-GGAGGATCCATGGCCTGGTTCTCAGC-3' (SEQ.ID.NO.:1; 5' oligo) 5'-CACAAGCTTAGRCCRTCC MG RCA RTTCCA-3' (SEQ.ID.NO.: 2; 3' oligo) where R=A or G, and M=A or C.
A 537 bp PCR fragment containing nucleotide 24 through to the middle of transmembrane 3 was digested with Bam HI and Hind III and was subcloned into a Bam HI-Hind III site of pBluescript.

### 2. Rat GPR 6 in situ probe

The in situ probe DNA fragment of rat GPR6 was obtained by PCR based on the published rat GPR6 cDNA sequences. The sequences of the oligonucleotides utilized were as follows:
5'-GGAGAAGCTTCTGGCGGCGATGAACGCTAG-3' (SEQ.ID.NO.: 3; 5' oligo)
5'-ACAGGATCCAGGTGGCTGCTAGCAAGAG-3' (SEQ.ID.NO.: 4; 3' oligo)
A 608 bp PCR fragment containing nucleotide -10 through to the middle of transmembrane domain 4 was digested with Bam HI and Hind III and was subcloned into Bam HI-Hind III site of pBluescript.

### 3. Rat GPR12 in situ probe

The in situ probe DNA fragment of rat GPR12 was obtained by PCR based on the published rat GPR12 cDNA sequences. The sequences of the oligonucleotides utilized were as follows:
5'-CTTAAGCTTAAAATGAACGAAGACCCGAAG-3' (SEQ.ID.NO.: 5; 5' oligo)
5'-GGAGGATCCCCAGAGCATCACTAGCAT-3' (SEQ.ID.NO.: 6; 3' oligo)
A 516 bp PCR fragment containing nucleotide -5 through to the middle of transmembrane domain 4 was digested with Bam HI and Hind III and subcloned into a Bam HI-Hind III site of pBluescript.

In situ probe sequences generated were as follows:

### Example 2

### Receptor Expression

### 1. cDNA and Vectors

With respect to GPR3 and GPR6, expression vectors comprising cDNA were generously supplied by Brian O'Dowd (University of Toronto). The vector for GPR3 cDNA was pcDNA3; the vector for GPR6 was pRcCMV (the coding region for GPR6 was subcloned into pCMV vector at a Hind III-Xbal site). GPR12 cDNA was prepared using the following protocol: Human GPR12 cDNA was obtained by PCR using human genomic DNA and a 5' primer from the 5' untranslated region with a Hind III restriction site, and a 3' primer from the 3' untranslated region containing a Bam HI site'. Primers had the following sequences:
5'-CTTAAGCTTGTGGCATTTGGTACT-3' (SEQ.ID.NO.: 10; 5' oligo)
5'-TCTGGATCCTTGGCCAGGCAGTGGAAGT-3 (SEQ.ID.NO.: 11; 3' oligo)
PCR was performed using rTth polymerase (Perkin Elmer) with the buffer system provided by the manufacturers, 0.25 µM of each primer, 0.2 µM of each of the four nucleotides and 0.2 µg of genomic DNA as template. The cycle condition was 30 cycles of 94°C for 1 min, 57 °C for 1 min and 72°C for 1.5 min. The 1.2 kb PCR fragment was digested with Hind III and Bam HI, and subcloned into Hind III-Bam HI site of pCMV expression vector. The resulting cDNA clones were fully sequenced and consistent with published sequences.

With respect to GPR21, PCR was performed using genomic DNA as template and rTth polymerase (Perkin Elmer) with the buffer system provided by the manufacturer, 0.25 µM of each primer, and 0.2 mM of each of the four nucleotides. The cycle condition was 30 cycles of 94°C for 1 min, 62°C for 1min and 72°C for 1 min and 20 sec. The 5' PCR primer was kinased with the sequence:
5'-GAGAATTCACTCCTGAGCTCAAGATGAACT-3' (SEQ.ID.NO.:12)
and the 3' primer contained a BamHI site with the sequence:
5'-CGGGATCCCCGTAACTGAGCCACTTCAGAT-3' (SEQ.ID.NO.:13).
The resulting 1.1 kb PCR fragment was digested with BamHI and cloned into EcoRV-BamHI site of pCMV expression vector. Nucleic acid (SEQ.ID.NO.:14) and amino acid (SEQ.ID.NO.:15) sequences for human GPR21 were thereafter determined.

With respect to AL022171, PCR was performed using genomic DNA as template and rTth polymerase (Perkin Elmer) with the buffer system provided by the manufacturer, 0.25 µM of each primer, and 0.2 mM of each of the four nucleotides. The cycle condition was 30 cycles of 94°C for 1 min, 54°C for 1min and 72 °C for 1 min and 20 sec. The 5' primer contains an HindIII site with the following sequence:
5'-AGGAAGCTTTAAATTTCCAAGCCATGAATG-3' (SEQ.ID.NO.:16)
and the 3' primer contained a EcoRI site with the following sequence:
5'-ACCGAATTCAGATTACATTTGATTTACTATG-3' (SEQ.ID.NO.: 17). The resulting 1.15 kb PCR fragment was digested with HindIII and EcoRI and cloned into HindIII-EcoRI site ofpCMV expression vector. Nucleic acid (SEQ.ID.NO.: 18) and amino acid (SEQ.ID.NO.: 19) sequences for human AL022171 were thereafter determined and verified.

With respect to GPR4 (GenBank accession number L36148), expression vectors comprising the cDNA was generously supplied by Brian O'Dowd (University of Toronto). The vector for GPR4 cDNA was pcDNA3 and this subcloned into pCMV vector at a Hind III-Xbal site (the 5' untranslated region between HindIII and an ApaI site was trimmed by conducting digestion/self ligation).

With respect to RE2 (GenBank accession number AF091890), PCR was performed using human brain cDNA as template and rTth polymerase (Perkin Elmer) with the buffer system provided by the manufacturer, 0.25 µM of each primer, and 0.2 mM of each of the four nucleotides. The cycle condition was 30 cycles of 94°C for 1 min, 62°C for 1min and 72°C for 1 min and 30 sec. The 5' PCR primer contained an EcoRI site with the sequence
5' -AGCGAATTCTGCCCACCCCACGCCGAGGTGCT-3' (SEQ. ID. No. 20)
and the 3' primer contained a BamHI site with the sequence
5'-TGCGGATCCGCCAGCTCTTGAGCCTGCACA-3' (SEQ.ID. NO.: 21). The 1.36 kb PCR fragment that resulted after two rounds of PCR was then digested with EcoRI and BamHI and cloned into EcoRI-BamHI site of pCMV. Nucleic acid (SEQ. ID. NO. 22) and amino acid sequence (SEQ. ID. NO. 23) was thereafter determined.

With respect to OGR1 (GenBank accession number U48405), PCR was performed using human genomic DNA as template and rTth polymerase (Perkin Elmer) with the buffer system provided by the manufacturer, 0.25 µM of each primer, and 0.2 mM of each of the four nucleotides. The cycle condition was 30 cycles of 94°C for 1 min, 62°C for 1min and 72°C for 1 min and 20 sec. The 5' PCR primer contained a HindIII site with the sequence 5'-GGAAGCTTCAGGCCCAAAGATGGGGAACAT-3' (SEQ. ID. No. 24)
and the 3' primer contain a BamHI site with the sequence
5'-GTGGATCCACCCGCGGAGGACCCAGGCTAG-3' (SEQ.ID.NO.25)_{.} The resulting 1.14 kb PCR fragment was digested with HindIII and BamHI and cloned into HindIII-BamHI site pCMV. Nucleic acid (SEQ. ID. NO. 26) and amino acid sequence (SEQ. ID. NO. 27) was thereafter determined.

With respect to GHSR, PCR was performed using hippocampus cDNA as template and TaqPlus Precision polymerase (Stratagene) with the buffer system provided by the manufacturer, 0.25 µM of each primer, and 0.2 mM of each 4 nucleotides. The cycle condition was 30 cycles of 94°C for 1 min, 68°C for 1min and 72°C for 1 min and 10 sec. For first round PCR, the 5' PCR primer sequence:
5'-ATGTGGAACGCGACGCCCAGCG-3' (SEQ.ID.NO.40)
and the 3' primer sequence:
5'-TCATGTATTAATACTAGATTCT-3' (SEQ.ID.NO.41).
Two microliters of the first round PCR was used as a template for the second round PCR
where the 5' primer was kinased with sequence:
5'-TACCATGTGGAACGCGACGCCCAGCGAAGAGCCGGGGT-3' (SEQ.ID.NO.:42) and the 3' primer contains an EcoRI site with the sequence:
5'-CGGAATTCATGTATTAATACTAGATTCTGTCCAGGCCCG-3' (SEQ.ID.NO.:43). The 1.1 kb PCR fragment was digested with EcoRI and cloned into blunt-EcoRI site of CMVp expression vector. Nucleic acid (SEQ.ID.NO.:44) and amino acid (SEQ.ID.NO.:45) sequences for human GHSR were thereafter determined.

### 2. Transfection procedure

On day one, 1X10⁷ 293 or 293T cells per 150mm plate were plated out. On day two, two reaction tubes were prepared (the proportions to follow for each tube are per plate): tube A was prepared by mixing between 8-20µg DNA (e.g., pCMV vector; pCMV vector with receptor cDNA; pCMV with GPCR Fusion Protein, *supra*) in 1-2ml serum free DMEM (Irvine Scientific, Irvine, CA); tube B was prepared by mixing 50-120µl lipofectamine (Gibco BRL) in 1-2ml serum free DMEM. Tubes A and B were then admixed by inversions (several times), followed by incubation at room temperature for 30-45min. The admixture is referred to as the "transfection mixture". Plated cells were washed with 1XPBS, followed by addition of 10-12ml serum free DMEM. 2.4ml of the transfection mixture was then added to the cells, followed by incubation for 4hrs at 37°C/5% CO₂. The transfection mixture was then removed by aspiration, followed by the addition of 25ml of DMEM/10% Fetal Bovine Serum. Cells were then incubated at 37°C/5% CO₂.

For GPCR Fusion Protein, preferred amounts to the above are as follows: 12µg DNA; 2ml serum free DMEM; 60µl lipofectamine; 293 cells 9 and an addition of 12ml serum free DMEM).

### Example 3

### Tissue Distribution of GPCR

For some orphan receptors, it will be apparent to those in the art that there is an understanding of the distribution of such receptors within, *e*.*g*., a human, or associated with a disease state. However, for many orphan receptors, such information is not known, or will not be known. It is therefore preferred that some understanding of where such receptors may be distributed be understood; this allows for the ability to gain a predictive opportunity to associate a particular receptor with a disease state or disorder associated with the particular tissue where the receptor may be preferentially expressed. Using a commercially available mRNA dot-blot format, the distribution of endogenous, constitutively active GPCRs in various tissue types was assessed.

Preferably, the entire coding region of the receptor is used to generate a radiolabeled probe using a Prime-It II™ Random Primer Labeling Kit (Stratagene, #300385), according to the manufacturer's instructions. As an example, this approach was utilized for GPR4.

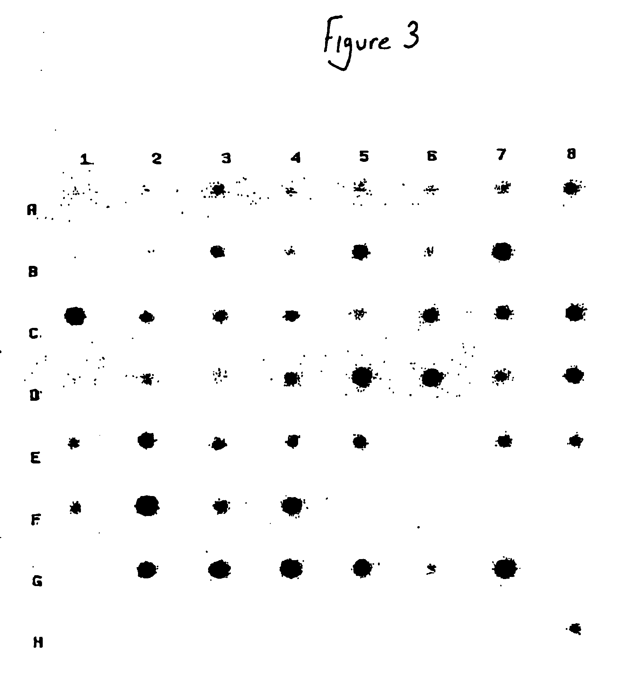

Human RNA Master Blot™ kit (Clontech, #7770-1) was hybridized with this probe and washed under stringent conditions, in accordance with manufacturer instructions. The blot was exposed to Kodak BioMax™ Autoradiography film overnight, at -80°C. Results are presented in Figure 3. Based upon these results, it is noted that GPR4 appears to be expressed throughout a variety of fetal tissue types (row G), as well as non-fetal heart (C 1), and non-fetal lung (F1). This approach can be readily utilized for other receptors.

### Example 4

### GTP MEMBRANE BINDING SCINTILLATION PROXIMITY ASSAY

When a G protein-coupled receptor is in its active state, either as a result of ligand binding or constitutive activation, the receptor binds to a G protein (in the case of GPR3, GPR4, GPR6, GPR12, GPR21, GHSR, OGR1, RE2 and AL022171, Gs) and stimulates the binding of GTP to the G protein. The trimeric G protein-receptor complex acts as a GTPase and slowly hydrolyzes the GTP to GDP, at which point the receptor normally is deactivated. Constitutively activated receptors continue to exchange GDP for GTP. The non-hydrolyzable GTP analog, [³⁵S]GTPγS, can be utilized to demonstrate enhanced binding of [³⁵S]GTPγS to membranes expressing constitutively activated receptors. The advantage of using [³⁵S]GTPyS binding to measure constitutive activation is that: (a) it is generically applicable to all G protein-coupled receptors; (b) it is proximal at the membrane surface making it less likely to pick-up molecules which affect the intracellular cascade.

The assay utilizes the ability of G protein coupled receptors to stimulate [³⁵S]GTPγS binding to membranes expressing the relevant receptors. The assay can, therefore, be used in the direct identification method to screen candidate compounds to known, orphan and constitutively activated G protein coupled receptors. The assay is generic and has application to drug discovery at all G protein coupled receptors.

The [³⁵S]GTPγS assay was incubated in 20 mM HEPES, pH 7.4, binding buffer with 12 nM [³⁵S]GTPγS and 75 µg membrane protein [e.g., 293T cells expressing GPR3] and 1 µM GDP for 1 hour. Wheatgerm agglutinin beads (25 µl; Amersham) were then added and the mixture was incubated for another 30 minutes at room temperature. The tubes were then centrifuged at 1500 X g for 5 minutes at room temperature and then counted in a scintillation counter.

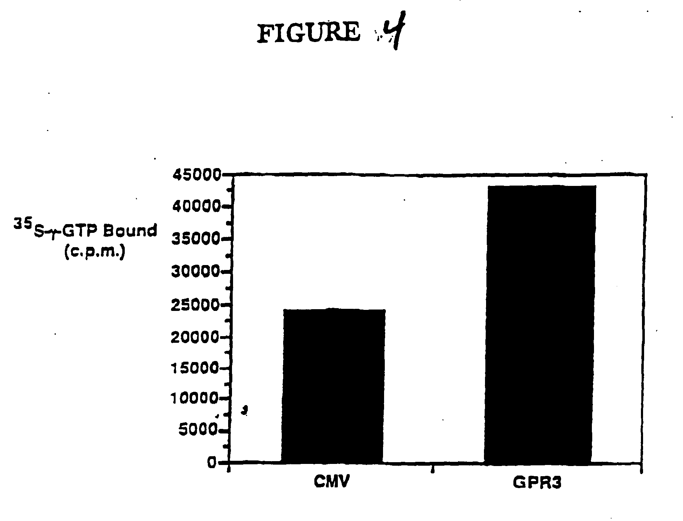

Referring to Figure 4, GPR3 receptor was determined to have increased activity as compared to control; this heightened activity is not the result of autocrine stimulation in that the data were obtained from membrane preparations, as opposed to whole cell preparations.

### Example 5

### Receptor Homology Determination

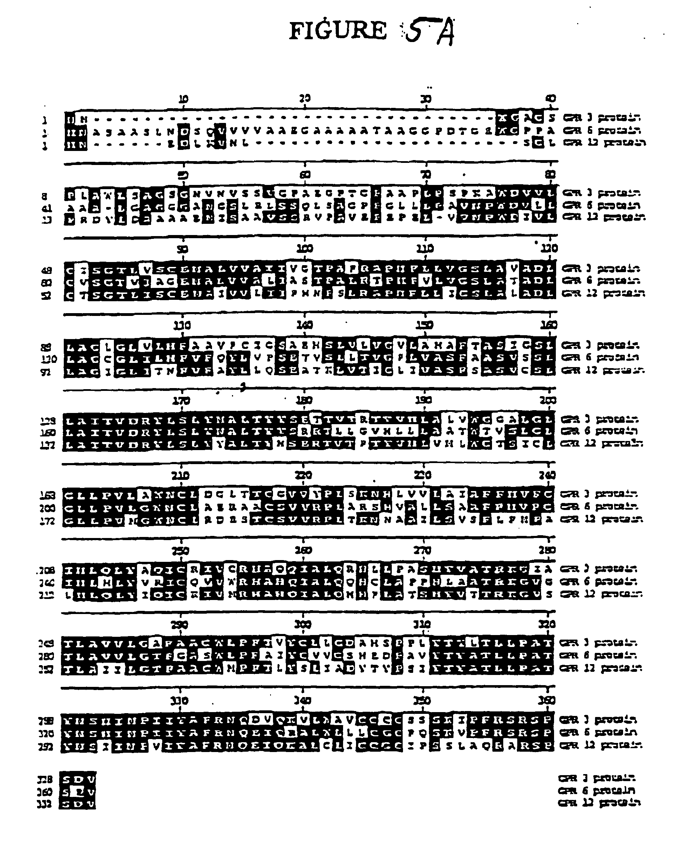

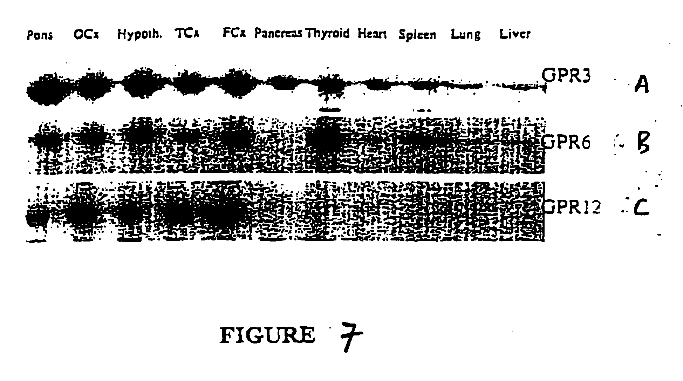

Following confirmation that GPR3 is a constitutively activated receptor, a homology search of the available G protein-coupled data banks (GeneBank), using the commercially available program, *DNA Star,* identified two highly homologous receptors, GPR6 and GPR12 (*see* Figure 5A); both of these receptors are orphan receptors. While the sequence of these receptors was previously "known" (i.e., they were available on the databases), it was not known that these two receptors are constitutively activated in their endogenous forms *(see* Example 6, Figure 7). Furthermore, heretofore there would be no reason to search for such receptors for use in a drug discovery program in that the ligands therefore are not known or have not been identified. As such, the dogma approach to drug discovery would at best find the homology between GPR3, GPR6 and GPR12 of minor interest or, more likely, irrelevant.

### Example 6

### Analysis of Homologous Receptors For Constitutive Activation

Although a variety of cells are available to the art for the expression of proteins, it is most preferred that mammalian cells be utilized. The primary reason for this is predicated upon practicalities, *i*.*e*., utilization of, *e.g.,* yeast cells for the expression of a GPCR, while possible, introduces into the protocol a non-mammalian cell which may not (indeed, in the case of yeast, does not) include the receptor-coupling, genetic-mechanism and secretary pathways that have evolved for mammalian systems - thus, results obtained in non-mammalian cells, while of potential use, are not as preferred as that obtained from mammalian cells. Of the mammalian cells, COS-7, 293 and 293T cells are particularly preferred, although the specific mammalian cell utilized can be predicated upon the particular needs of the artisan.

### 1. Analysis of GPR3, GPR6 and GPR12

To generate a β-galactosidase reporter containing multiple Gal4 binding sites, a Bgl II/ HindIII fragment was removed from the somatostatin promoter-containing plasmid 1.4(5xGal)CAT (Leonard, J. et al (1992) PNAS USA 89:6247-6251) and cloned into p β gal-Basic (Promega). The Bgl II/ HindIII fragment contains a variant of the minimal somatostatin promoter (from -71 bp to +50 bp relative to the transcription start site) in which the core 4bp of the cAMP Response Element (-46 to -43) were replaced with 5 copies of the recognition sequence for the yeast transcription factor Gal4. When this reporter is co-transfected with an expression plasmid encoding a Gal4-CREB fusion protein, it is highly responsive to agents that increase the cAMP signaling pathway.

VIP2.0Zc is a cell line that has been stably transfected with the reporter gene β-galactosidase under the control of a cAMP responsive VIP promoter (Konig et al. Mol. Cell.Neuro. 1991, 2, 331-337). The cell line was used here to indirectly measure the accumulation of intracellular cAMP. Approximately 2 million cells were plated in 6 cm plate the day before transfection. DNA (5 µg), for each receptor, was mixed with 2.5 ml serum-free DMEM containing 200 µg/ml DEAE dextran and 100 µM chloroquine, and added to a rinsed cell monolayer. After incubation for 90 min in a CO₂ incubator, the transfection medium was removed. The cells were washed with serum-free medium and supplemented with fresh complete medium. Twenty four hours after transfection, the cells were replated into 96-well plate at a density of 50 - 100 K per well and the β-galactosidase activity was assayed 48 to 72 hours after transfection.

The assay buffer contained 100 mM sodium phosphate, 2 mM MgSO₄, 0.1 mM MnCl₂, pH 8.0. The cells were washed with PBS, and 25 µl /well of hypotonic lysis buffer consisting of 0.1 X assay buffer was added. Ten minutes later, 100 µl of assay buffer containing 0.5% Triton X-100 and 40 mM β-mercaptoethanol was added to each well and incubation at room temperature continued for 10 minutes. The substrate solution containing 5 mg/ml chlorophenol red- β-D-galactopyranoside (CPRG) in assay buffer was added at 25 µl/well and the plate was incubated at 37°C for 30 minutes before absorbance at 595 nm was measured with a plate reader.

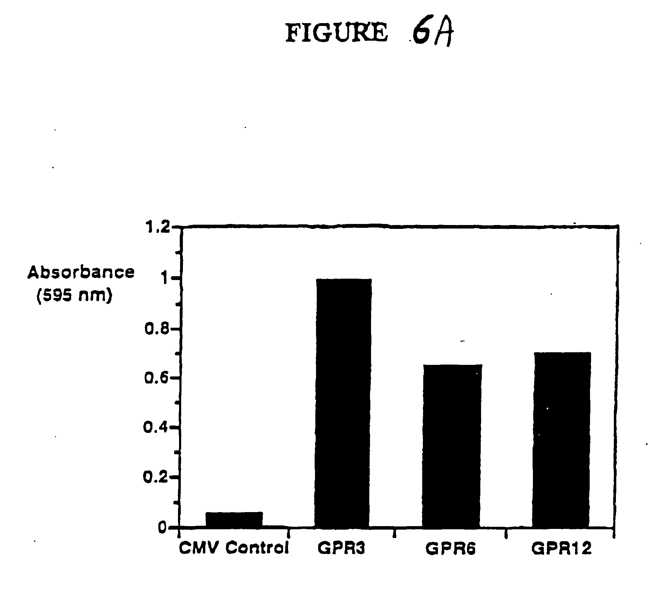

GPR3, GPR6 and GPR 12 were assayed using the foregoing system, and it was determined that both GPR6 and GPR12 are constitutively active. *See* Figure 6A.

### 2. Analysis of GPR21 and AL022171

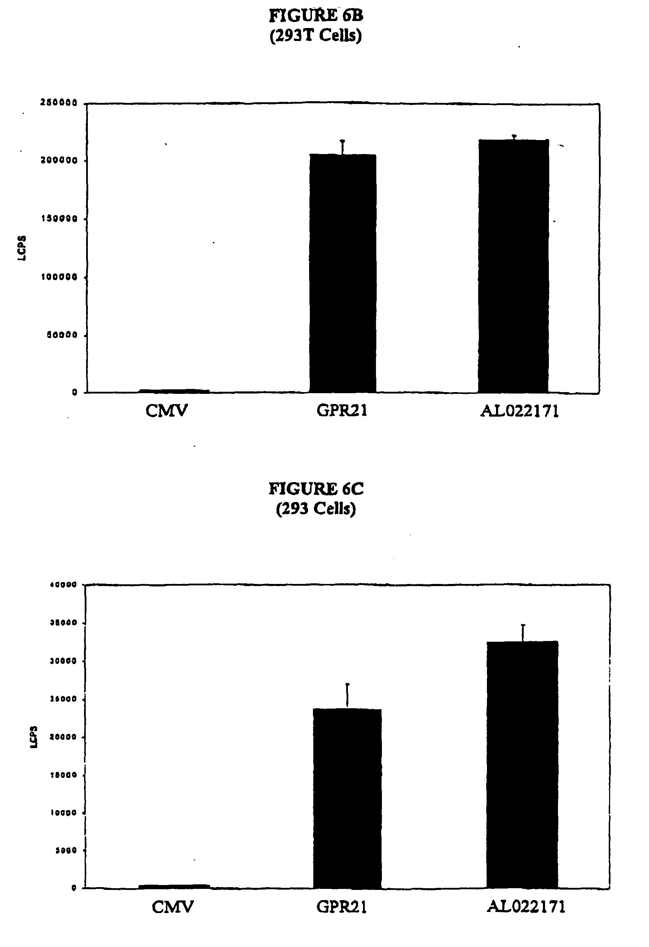

293 and 293T cells were plated-out on 96 well plates at a density of 2 x 10⁴ cells per well and were transfected, using Lipofectamine Reagent (BRL), the following day according to manufacturer instructions. A DNA/lipid mixture was prepared for each 6-well transfection as follows: 260ng of plasmid DNA in 100µl of DMEM were gently mixed with 2µl of lipid in 100µl of DMEM (the 260ng of plasmid DNA consisted of 200ng of a 8xCRE-Luc reporter plasmid, 50ng of pCMV comprising endogenous receptor or non-endogenous receptor or pCMV alone, and 10ng of a GPRS expression plasmid (GPRS in pcDNA3 (Invitrogen)). The 8XCRE-Luc reporter plasmid was prepared as follows: vector SRIF-β-gal was obtained by cloning the rat somatostatin promoter (-71/+51) at BgIV-HindIII site in the pβgal-Basic Vector (Clontech). Eight (8) copies of cAMP response element were obtained by PCR from an adenovirus template AdpCF126CCRE8 (*see* 7 Human Gene Therapy 1883 (1996)) and cloned into the SRIF-β-gal vector at the Kpn-BgIV site, resulting in the 8xCRE-β-gal reporter vector. The 8xCRE-Luc reporter plasmid was generated by replacing the beta-galactosidase gene in the 8xCRE-β-gal reporter vector with the luciferase gene obtained from the pGL3-basic vector (Promega) at the HindIII-BamHI site. Following 30 min. incubation at room temperature, the DNA/lipid mixture was diluted with 400 µl of DMEM and 100µl of the diluted mixture was added to each well. 100 µl of DMEM with 10% FCS were added to each well after a 4hr incubation in a cell culture incubator. The following day the transfected cells were changed with 200 µl/well of DMEM with 10% FCS. Eight (8) hours later, the wells were changed to 100 µl /well of DMEM without phenol red, after one wash with PBS. Luciferase activity were measured the next day using the LucLite™ reporter gene assay kit (Packard) following manufacturer instructions and read on a 1450 MicroBeta™ scintillation and luminescence counter (Wallac). Results are summarized in Figures 6B and 6C.

GPR21 and AL022171 were assayed using the foregoing system, and based upon these results, it was determined that both GPR21 and AL022171 are constitutively active in their endogenous forms. *See* Figure 6B and 6C.

### 3. Analysis of GPR4, RE2, OGR1 and GHSR

Using the protocols defined herein, GPR4, RE2, OGR1 and GHSR were analyzed and determined to be constitutively active in their endogenous forms (data not shown).

### Example 7

### Tissue Distribution of GPR3, GPR6 and GPR12

Tissue samples were examined for expression of these orphan receptors by comparative RT-PCR, using the following primers:
**GPR3:**
   5'-CTGGTCCTGCACTTTGCTGC-3' (SEQ. ID. NO.: 28)
   5'-AGCATCACATAGGTCCGTGTCAC-3' (SEQ.ID.NO.: 29)
   These primers amplify a 194bp fragment.
**GPR6:**
   5'-ACCAGAAAGGGTGTGGGTACACTG-3' (SEQ. ID. NO.: 30)
   5'-GGAACGAAAGGGCACTTTGG-3' (SEQ. ID. NO.: 31)
   These primers amplify a 249bp fragment.
**GPR12:**
   5'-GCTGCCTCGGGATTATTTAG-3' (SEQ. ID. NO.: 32)
   5'-GCCTATTAGCAGGAACATGGGTG-3' (SEQ. ID. NO.: 33)
   These primers amplify a 220bp fragment.

These amplicons were designed to be non-overlapping, i.e., there is no sequence similarity between them, and to have similar Tm's, such that each primer pair amplifies its respective target at the same optimal annealing temperature. This diminishes the chance that an amplicon from one primer pair will act as an annealing target for the other primers in the multiplex reaction, therefore reducing the chance of interference with other primer pairs.

Total RNA was extracted from tissue samples (human) using TRIzol™ Reagent (Gibco/BRL), following manufacturer instructions. cDNA was generated using 2mg total RNA and a First-Strand™ cDNA synthesis kit (Pharmacia). The cDNA samples were then diluted 1:3 in H₂O and comparative PCR was performed as described (Jensen, J. et al. (1996) J. Biol. Chem. 271:187490) in the presence of [³²P]dCTP. All reactions included the SP 1-specific primers, which amplify a 300bp fragment, to serve as an internal control. Using the primers outlined above, under defined PCR conditions (1 cycle: 95°C, 5min; 23 cycles: 95°C, 30sec, 58°C, 30sec, 72°C, 1min; 1 cycle: 72°C, 10min) gave consistently reliable and quantitatively accurate results. It was further determined that the selected primer pairs did not interfere with each other when multiplexed. PCR products were visualized by denaturing gel electrophoresis (7M urea, 5% polyacrylamide (Long Ranger™ Solution, AT Biochemical, 0.6 XTBE) and subsequent autoradiography.

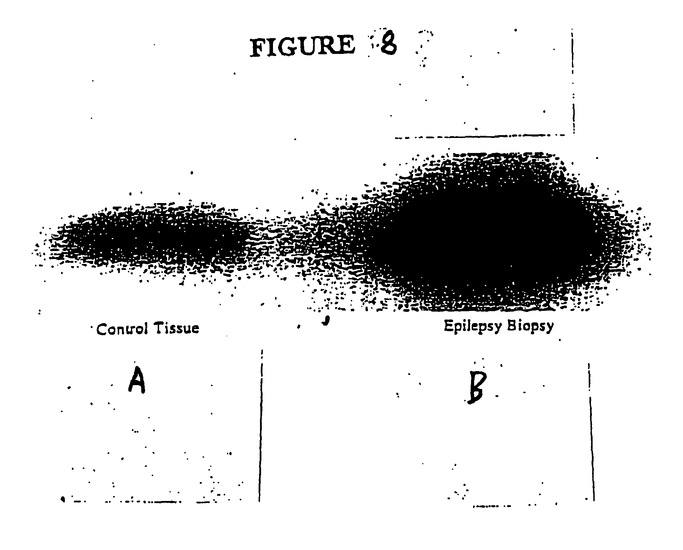

Figures 7A, 7B, and 7C show the distribution of GPR3, GPR6 and GPR12 across human tissues. This information allows for assessing disease states that are associated with such tissue, as well as determining specific regions within such tissue where such expression predominates, thus allowing for correlating such receptor expression with particular disease states. This, in tum, then allows for direct identification of compounds that impact such receptors, without the need to understand or know the endogenous ligand for such receptor. Further screening reveals that GPR3 is expressed in much higher levels in human epilepsy tissue samples (tissue source: temporal cortex), as compared with controls, as evidenced by RT-PCR analysis (Figure 8).

### Example 8A

### Functional Analysis - GPR6 (In Situ Analysis)

The distribution of GPR6 in the hypothalamus suggested possible involvement in feeding behavior. Accordingly, a functional analysis of this receptor was undertaken. In situ analysis was conducted as follows:

### 1. Probe Design

GPR6 probe was produced from a 450bp HindIII-ScaI fragment of the GPR6 receptor cloned into the HindIII-SmaI site of pBluescriptSK+. Riboprobes were produced using a T7 transcription system in a standard labeling reaction consisting of: 1µg of linearized plasmid, 2µl of 5x transcription buffer, 125µCi of ³⁵S-UTP, 150µM of GTP, CTP and ATP, 12.5mM dithiothreitol, 20U of RNase inhibitor and 6U of appropriate polymerase. The reaction was incubated at 37°C for 90 min., labeled probe being separated from free nucleotides over Sephadex G-50 spin columns.

### 2. Tissue preparation

Dissected tissue was frozen in isopentane cooled to -42°C and subsequently stored at -80°C prior to sectioning on a cryostat maintained at -20°C. Slide-mounted tissue sections were then stored at -80 °C.

### 3. In Situ Hybridization Protocol

Tissue sections were removed from the -80°C freezer and incubated with a 1 µg/ml solution of proteinase-K to permeabilize the tissue and inactivate endogenous RNase. After this treatment, sections were incubated in succession in water (1 min), 0.1 M triethanolamine (pH 8.0; 1 min), and 0.25% acetic anhydride in 0.1 M triethanolamine (10 min). The tissue was then washed in 2 x SSC (0.3 mM NaCl, 0.03 nM Na citrate, pH 7.2; 5 min) and dehydrated through graded concentrations of ethanol. Sections were then hybridized with 1.5 x 10⁶ dpm of [³⁵S]UTP-labeled cRNA probes in 20 µl of a hybridization buffer containing 75% formamide, 10% dextran sulfate, 3 x SSC, 50 mM sodium phosphate buffer (pH 7.4), 1 x Denhart's solution, 0.1 mg/ml yeast tRNA, and 0.1 mg/ml sheared salmon sperm DNA. Tissue sections were covered with coverslips that were sealed with rubber cement. The slides were incubated overnight at 50°C. On the following day, the rubber cement was removed, the coverslips were soaked-off with 2 x SSC, and the tissue sections were washed for 10 min in fresh 2 x SSC solution. Single stranded probe not hybridized with endogenous mRNAs was removed by incubating the sections for 30 min in 200 µg/ml solution of RNase-A at 37°C. The tissue was then washed in increasingly stringent SSC solutions (2, 1 and 0.5 x SSC; 10 min each), followed by a 1 hr wash in 0.5 x SSC at 60°C. After this final wash, the tissue sections were dehydrated using graded concentrations of ethanol, air dried and prepared for detection by x-ray autoradiography on Kodak XAR-5 film.

### 4. Analysis

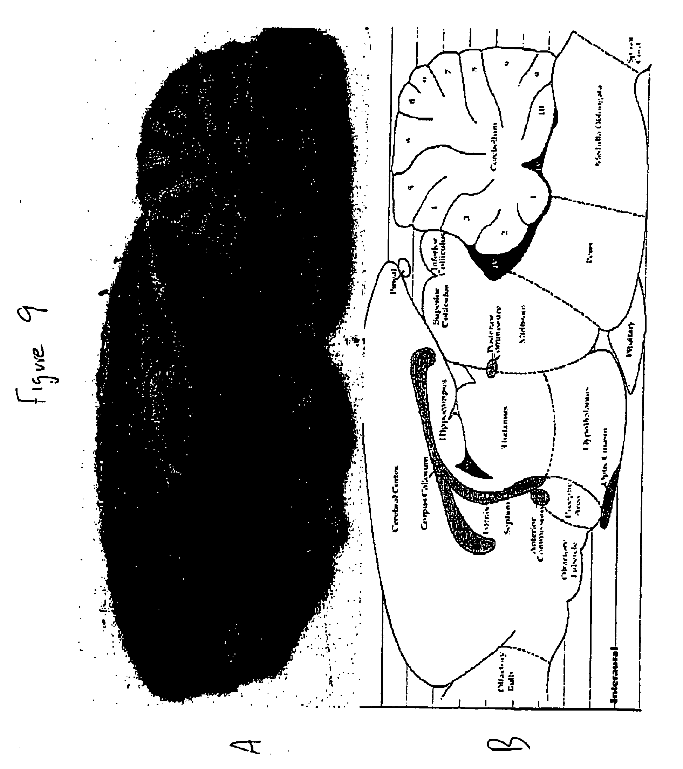

Utilizing the above protocol on normal male rats (Sprague-Dawley, Charles River), it was determined that GPR6 is expressed in the following areas of the brain: hypothalamus, hippocampus, nucleus accumbens, caudate and cerebral cortex. *See* Figure 9A for a representative tissue section (GPR6 receptor is presented in the dark areas; Figure 9B provides a reference map of the rat brain.)

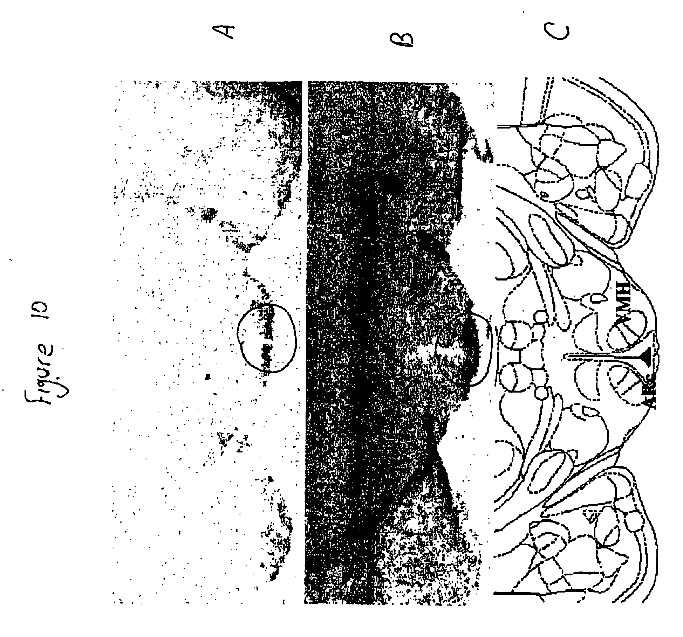

Given the high levels of expression of GPR6 in areas of the brain associated with feeding, an in situ analysis was conducted using the above protocol on both lean and obese male Zucker rats (Charles River). As those in the art appreciate, the Zucker animals are genetically bred to result in animals that exhibit a lean or obese phenotype. Figure 10A provides a representative tissue section of GPR6 receptor expression in the lean Zucker animals; Figure 10B provides a representative tissue section of GPR6 receptor expression in the obese Zucker animals; Figure 10C is a reference map of this section of the rat brain. These results support the position that the endogenous, constitutively activated orphan receptor GPR6 is relatively overexpressed in a model of obesity.

### Example 8B

### Functional Analysis - GPR12 (In Situ Analysis)

In situ analysis for the GPR12 receptor was conducted as follows:

### 1. Probe Design

GPR12 probe was produced from a 515bp (NT5 - NT520) HindIII-BamHI fragment of the rat GPR12 receptor cloned into the HindIII-BamHI site of pBluescriptSK+. Riboprobes were produced using a T3/T7 transcription system in a standard labeling reaction consisting of 1µg of linearized plasmid, 2µl of 5x transcription buffer, 125µCi of ³⁵S-UTP, 150µM of GTP, CTP and ATP, 12.5mM dithiothreitol, 20U of Rnase inhibitor and 6U of appropriate polymerase. The reaction was incubated at 37°C for 90 min., labeled probe being separated from free nucleotides over Sephadex G-50 spin columns.

### 2. Tissue preparation

Dissected tissue was frozen in isopentane cooled to -42°C and subsequently stored at -80°C prior to sectioning on a cryostat maintained at -20°C. Slide-mounted tissue sections were then stored at -80 °C.

### 3. In Situ Hybridization Protocol

Tissue sections were removed from the -80°C freezer and incubated with a 1 µg/ml solution of proteinase-K to permeabilize the tissue and inactivate endogenous RNase. After this treatment, sections are incubated in succession in water (1 min), 0.1 M triethanolamine (pH 8.0; 1 min), and 0.25% acetic anhydride in 0.1 M triethanolamine (10 min). The tissue was then washed in 2 x SSC (0.3 mM NaCl, 0.03 nM Na citrate, pH 7.2; 5 min) and dehydrated through graded concentrations of ethanol. Sections were then hybridized with 1.5 x 10⁶ dpm of [³⁵S]UTP-labeled cRNA probes in 20 µl of a hybridization buffer containing 75% formamide, 10% dextran sulfate, 3 x SSC, 50 mM sodium phosphate buffer (pH 7.4), 1 x Denhart's solution, 0.1 mg/ml yeast tRNA, and 0.1 mg/ml sheared salmon sperm DNA. Tissue sections were covered with coverslips that were sealed with rubber cement. The slides were incubated overnight at 50°C. On the following day, the rubber cement was removed, the coverslips were soaked-offwith 2 x SSC, and the tissue sections were washed for 10 min in fresh 2 x SSC solution. Single stranded probe not hybridized with endogenous mRNAs was removed by incubating the sections for 30 min in 200 µg/ml solution of RNase-A at 37°C. The tissue was then washed in increasingly stringent SSC solutions (2, 1 and 0.5 x SSC; 10 min each), followed by a 1 hr wash in 0.5 x SSC at 60°C. After this final wash, the tissue sections were dehydrated using graded concentrations of ethanol, air dried and prepared for detection by x-ray autoradiography on Kodak XAR-5 film.

### 4. Analysis

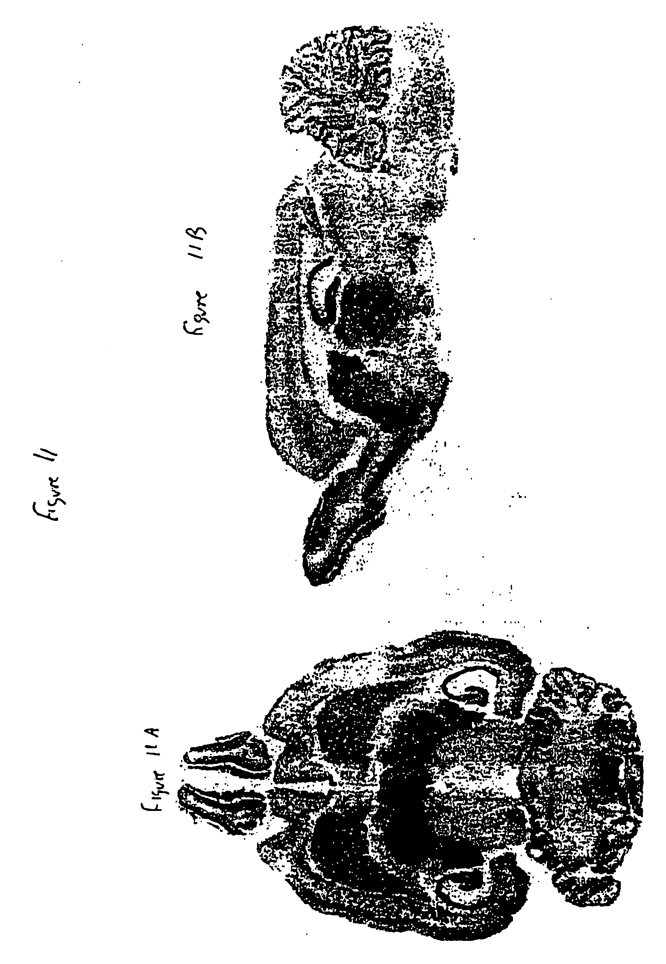

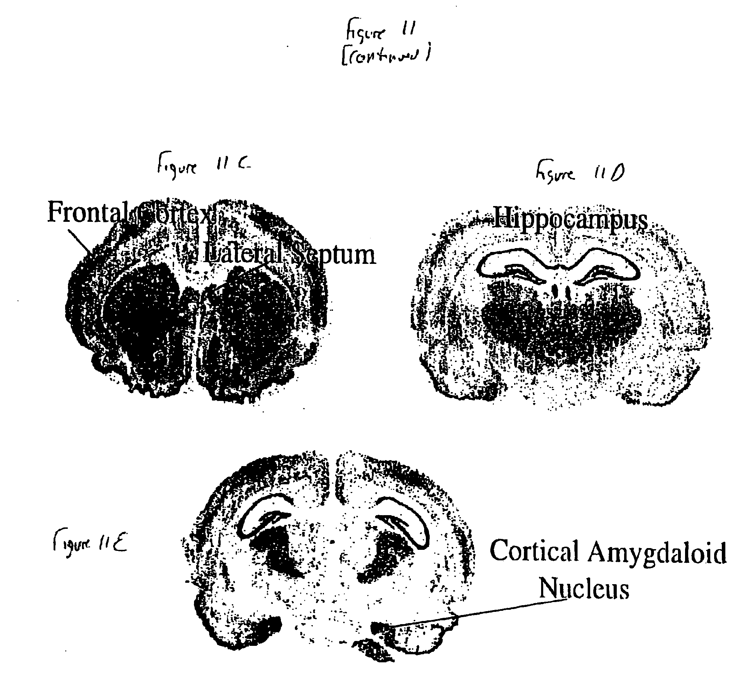

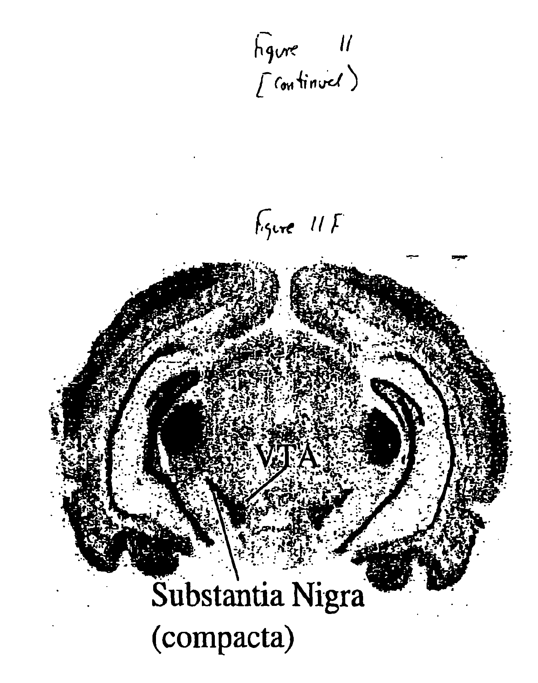

Utilizing the above protocol on normal male rats (Sprague-Dawley, Charles River), it was determined that GPR12 is expressed in the following areas of the brain: hippocampus (particularly in regions CA₃, CA₄ and the dentate gyrus; outer layers of the cerebral cortex; and the amygdala - all of these regions are well known in the art as associated with regions important for learning and memory); and thalamic relay nuclei, including the lateral geniculate nucleus, the medial geniculate nucleus and the lateral thelamic nucleus (regions related to lateral relay functions, e.g., vision and hearing). *See* Figures 11A-F for representative tissue sections (GPR12 receptor is presented in the dark areas).

### Example 8C

### Functional Analysis - Co-Localization of GPR6 With Feeding-Behavior Receptors (In Situ Analysis)

The human orexin receptor OX₁R, previously an orphan GPCR (a.k.a., "HFGAN72"), has been localized in the lateral hypothalamic region of the brain and has been hypothesized to be involved in regulation of feeding behavior. Sakurai, T. et al 92(4) Cell 573 (1998). As noted in Sakurai, "pharmacological intervention directed at the orexin receptors may prove to be an attractive avenue toward the discovery of novel therapeutics for diseases involving disregulation of energy homeostasis, such as obesity and diabetes mellitus." *Id* at 582. The melanocortin-3 receptor (MC-3) has also been identified, Gantz, I. Et al, 268(11) J. Biol. Chem. 8246 (1993), and is similarly associated with energy homeostasis.

An understanding of the neural pathways involved in the regulation and disregulation of energy homeostasis is important for appreciation of hierarchical nuances that are critical for rational drug design. Merely affecting one receptor, particularly a receptor that is "downstream" of a more relevant receptor-pathway, may lead to a substantial expenditure of time and resources that ultimately results in the development of a pharmaceutical compound that may have little, if any, substantive impact on a particular disease state. For example, leptin, while clearly involved in some fashion with energy homeostasis, has not, to date, evidenced an opportunity for the development of a pharmaceutical product in the area of obesity. And while both the OX₁and MC-3 receptors (as well as other melanocortin receptors) are also, in some manner, involved with energy homeostasis, development of pharmaceuticals based upon the traditional receptor "antagonist" approach may prove to be more frustrating than fruitful if, for example, these receptors are not constitutively active in their endogenous forms, and, within the energy homeostasis pathway, there is a receptor that is constitutively active in its endogenous state. Indeed, the endogenous, constitutively active receptor would, by definition, continually signal whereas the endogenous, non-constitutively active receptors would require ligand-binding for such signaling. Thus, in the case of GPR6, which is not only constitutively active in its endogenous form, but also appears to be significantly up-regulated in an animal model of obesity, GPR6 would, in essence, "trump" other energy homeostasis related receptors in that even with complete blockage via receptor antagonists to these receptors, GPR6 would continue signaling. Thus, a determination of whether these receptors (and others within the energy homeostasis pathway) are co-localized within discrete, neuronal regions, is useful in providing a more refined receptor target for drug development.

In situ hybridization studies were performed as described above for GPR6, OX₁R and MC-3 receptors. For GPR6, the in situ probe utilized was as set forth in Example 7A. For OX₁R and MC-3, the probes were based upon the published rat sequences and were approximately 950bp and 441bp, respectively. Tissue preparation (normal rats) and in situ hybridization were substantially the same as set forth in Example 8A.

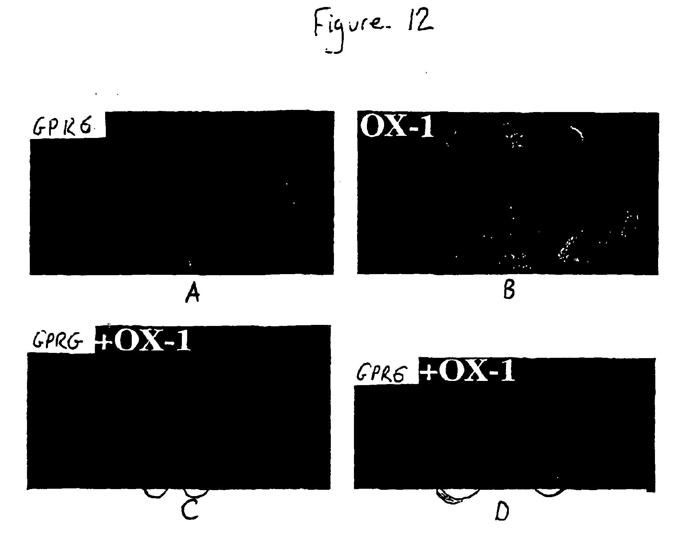

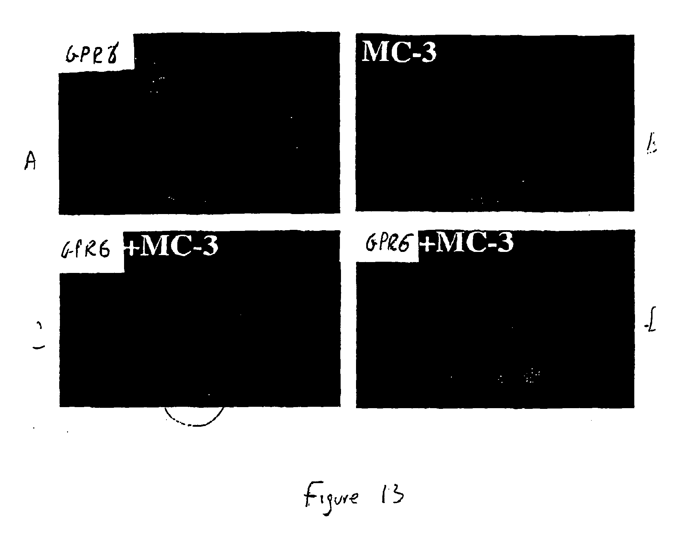

Results are presented in Figure 12 (GPR6 and OX₁R) and Figure 13 (GPR6 and MC-3), where a red filter was used for GPR6 hybridization and a green filter was used for OX₁R (Figure 12B) and MC-3 (Figure 13B). Figures 12C and 12D (a magnified version of 12A) are generated by overlay of Figures 12A and 12B; co-localization is evidenced by areas having an orange color (from the combination of red and green). Thus, in Figure 12C, it can be seen that GPR6 and OX₁R are co-localized in a sub-set of cells in the lateral arcuate and in the ventromedial hypothalamic nucleus, both of these regions being involved in the energy homeostasis pathways. A similar overlay-procedure for Figures 13A (GPR6) and 13B (MC-3) provides evidence that these receptors are co-localized primarily in the lateral arcuate.

Information continues to develop within the art as to the neural pathways associated with feeding behavior. An important component of this pathway is the neuropeptide agouti-related peptide (AGRP) sometimes referred to as agouti-related transcript (ART). The expression of AGRP is largely restricted to the arcuate nucleus *(see* Flier, J. S. and Maratos-Flier, E., and Figure 1 therein ). The cells that produce AGRP also produce neuropeptide Y (NPY). Animal studies have evidenced that administration of AGRP and administration of NPY leads to increases in feeding behavior and obesity. AGRP has also been shown to be an antagonist to the melanocortin 4 (MC-4) receptor, and antagonism of the MC-4 receptor is also known to increase feeding behavior an obesity. Thus, AGRP appears to be involved in at least two pathways associated with feeding behavior. As set forth below, it has been discovered that the GPR6 receptor is co-localized within cells that produce AGRP, and based upon the results set forth below in Example 8, coupled with the fact that GPR6 is an endogenous, constitutively activated GPCR, it is apparent that GPR6 is in some manner a potential "regulator" of the system - when expression of the GPR6 receptor is reduced via the use of antisense protocols (Example 9) there was a exceedingly rapid loss in body weight of the animals tested, suggesting that GPR6 may regulate the expression of AGRP.

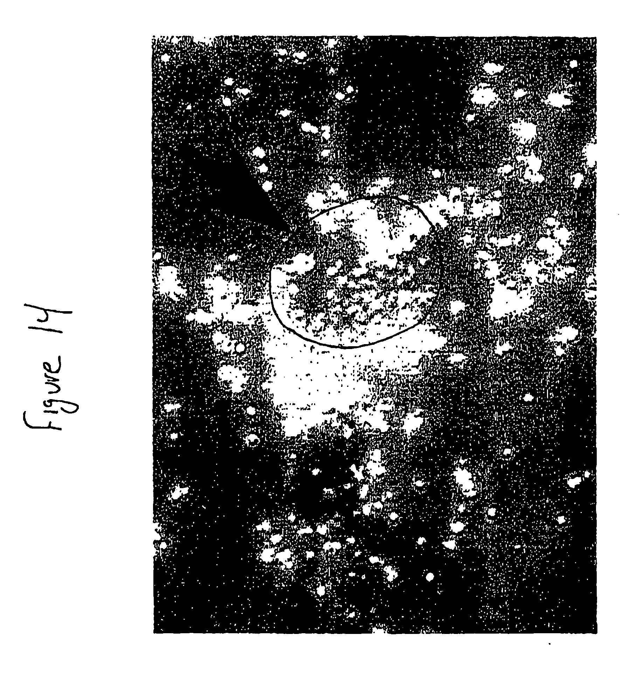

Unlike the "overlay" approach above, the protocol set forth in Marks, D.L. et al, 3 Mol. & Cell. Neuro. 395 (1992) was utilized for assessment of co-localization. AGRP (the AGRP cRNA probe was synthesized from a 382bp fragment of AGRP cDNA cloned into Bluescript SK vector) was analyzed in conjunction with radiolabeled GPR6 and both were found to be co-localized in the arcuate (*see* Figure 14). Given the role that AGRP plays with respect to homeostasis, and further given hat GPR6 is constitutively active in its endogenous state, the results obtained from Example 9, *infra,* would be consistent with these data in that the almost immediate, significant loss of weight can be understood in the context of GPR6 influencing AGRP.

### Example 9

### Functional Analysis - GPR6 (In Vivo Analysis: GPR6 Antisense)

Based upon the results developed from Example 7, and while not wishing to be bound by any particular theory, it was hypothesized that reduction in the expression of the GPR6 receptor would lead to a reduction in, *inter alia*, feeding behavior, metabolism, body weight, etc.; thus, by decreasing expression of this receptor via use of an antisense oligonucleotide, it was hypothesized that such animals would evidence changes in functional feeding behavior and/or feeding-related metabolism. Examination ofthis hypothesis was considered analogous to utilization of an inverse agonist to the receptor in that an inverse agonist would reduce the constitutive activity of the GPR6 receptor, akin to reducing the expression of the receptor itself. It is noted that such an approach results in "knock-down", as opposed to "knock-out", of the receptor, *i*.*e*., in general, it is accepted that an antisense approach reduces expression of the target protein by approximately 30%.

Sixteen adult male Sprague-Dawley rats (Harlan, San Diego) were used for this study. Animals were vivarium-acclimated for at least one week prior to use. Animals were housed (groups of two) in hanging plastic cages with food and water available ad lib. Animals were weighed and handled for at least one day prior to surgery (to establish baseline weight) and throughout the study (to assess the effects of the treatment). Daily food intake for pairs of animals in a cage was assessed by weighing the food in the feeding trough each morning before and after refilling. Groups included antisense (n = 5), missense (n = 4) and sterile water (n = 5).

Surgeries were performed under sodium pentobarbital anesthesia (60 mg/kg), supplemented with halothane as necessary. Animals were stereotaxically implanted with a single cannula (brain infusion kit, Alza Pharmaceuticals) aimed at the lateral ventricle (bregma, AP -1.0, Lat -1.5, DV -3.8 from the surface of the brain). The inlet of the cannula was connected via flexible tubing to the outlet of an osmotic minipump (Model 2001, Alza Pharmaceuticals), that was implanted subcutaneously between the shoulder blades according to instructions provided by the manufacturer.

Pumps contained antisense oligonucleotide 5'-GsCTAGCGTTCATCGCCGsC-3' (SEQ.ID.NO.:34; antisense) (wherein the small "s" denotes a phosphorothioate linkage) or missense oligonucleotide 5'-CsTGGACTGTATCGCCCCsG-3' (SEQ.ID.NO.: 35; missense), or sterile water vehicle. Oligonucleotides were synthesized by Genset Corp and diluted to 2µg/µl in sterile water. Because the pumps utilized deliver 1 µl/hour, animals received 48µg/day of antisense or missense oligonucleotides, or 24µl/day of sterile water. Pumps were primed prior to implant by incubation in sterile saline at 37°C for at least four hours prior to implant.

Five days after surgery, animals were treated with d-amphetamine sulfate; six days after surgery, baseline and amphetamine-stimulated locomotor behavior were examined; seven days after surgery, animals were euthanized and brains rapidly removed and frozen for histological analysis.

Animals were taken from the vivarium to the testing room, placed into an open field enclosure (*see* below), and baseline activity assessed for 30 minutes. At the end of 30 minutes, animals were briefly removed from the enclosure, injected with d-amphetamine sulfate (1.0 mg/kg s.c., diluted in sterile saline; National Institute on Drug Abuse Drug Supply Program), and immediately returned to the enclosure for 150 minutes. Locomotor behavior was quantified at 10 minute intervals in order to follow the time-course of baseline and amphetamine-stimulated activity.

Baseline and amphetamine-stimulated locomotor behavior were assessed in a San Diego Instruments Flex Field System, consisting of 16" x 16" x 15" clear plexiglas open field enclosures. Photocell arrays (16 in each dimension) which surrounded the open fields were interfaced with a personal computer for collection of data. One array at 2" above the floor of an enclosure detected locomotor activity, and a second at 5" detected rearing behavior. The computer provided a variety of measures of locomotor activity, including total photocell beam breaks, time active, time resting, distance traveled, total number of rears, and time spent rearing (data not shown). During testing, the testing room was dimly lit by an overhead incandescent bulb, with white noise to mask outside sounds.

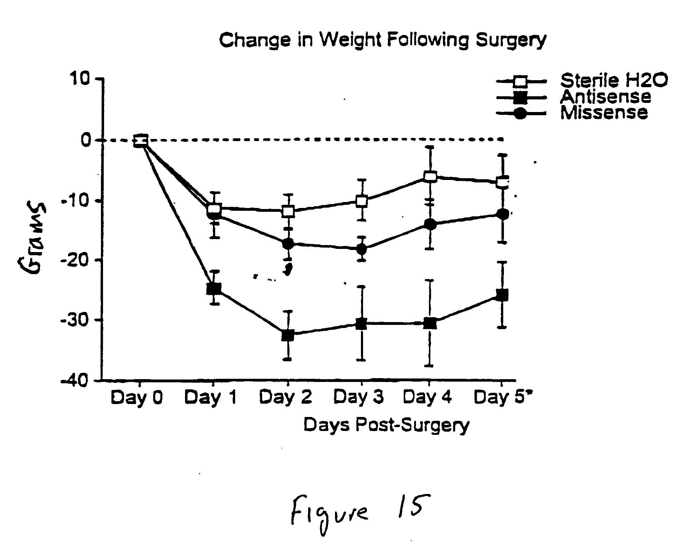

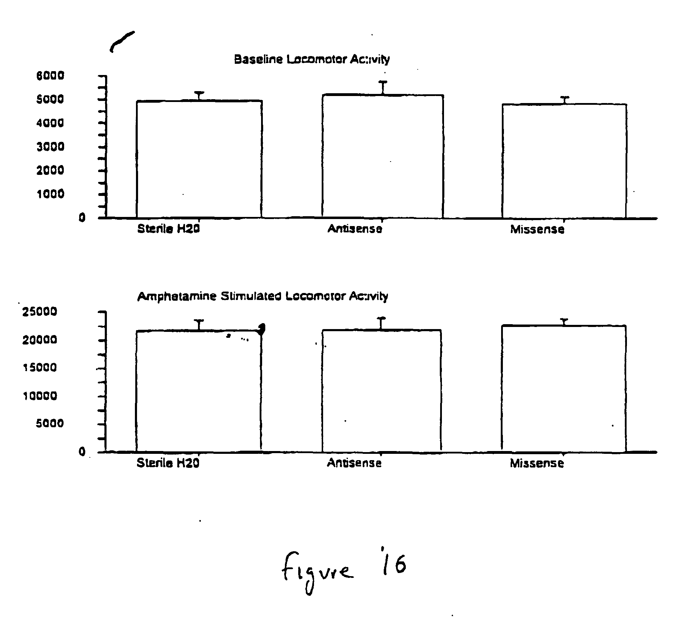

Results are presented in Figures 15 and 16. In Figure 15, it is noted that animals receiving the antisense oligonucleotide (GPR6 "knock-down" animals) had significantly greater loss of weight as compared with either the missense oligonucleotide-treated animals, or the control-treated animals. With respect to locomotor activity, the results of Figure 16 support the position that the base-line and amphetamine-treatment locomotor activities were substantially the same across all three groups.

### Example 10

### GPCR Fusion Protein Preparation

The design of the endogenous, constitutively activated GPCR-G protein fusion construct was accomplished as follows: both the 5' and 3' ends of the rat G protein Gsα (long form; Itoh, H. et al., 83 PNAS 3776 (1986)) were engineered to include a HindIII (5'-AAGCTT-3') sequence thereon. Following confirmation of the correct sequence (including the flanking HindIII sequences), the entire sequence was shuttled into pcDNA3.1(-) (Invitrogen, cat. no. V795-20) by subcloning using the HindIII restriction site of that vector. The correct orientation for the Gsα sequence was determined after subcloning into pcDNA3.1(-). The modified pcDNA3.1(-) containing the rat Gsα gene at HindIII sequence was then verified; this vector was now available as a "universal" Gsα protein vector. The pcDNA3.1(-) vector contains a variety of well-known restriction sites upstream of the HindIII site, thus beneficially providing the ability to insert, upstream of the Gs protein, the coding sequence of an endogenous, constitutively active GPCR. This same approach can be utilized to create other "universal" G protein vectors, and, of course, other commercially available or proprietary vectors known to the artisan can be utilized - the important criteria is that the sequence for the GPCR be upstream and in-frame with that of the G protein.

Both GPR3-Gsα Fusion Protein construct and GPR6-Gsα Fusion Protein construct were then made as follows: primers were designed for both the GPR3 and GPR6. For GPR3, the primers were as follows:
5'-gatcTCTAGAATGATGTGGGGTGCAGGCAGCC-3' (SEQ. ID. NO. 36; sense)
5'-ctagGGTACCCGGACATCACTGGGGGAGCGGGATC-3' (SEQ. ID. NO. 37, antisense)
The sense and anti-sense primers included the restriction sites for XbaI and KpnI, respectively. For GPR6, the primers were as follows:
5'-gatcTCTAGAATGCAGGGTGCAAATCCGGCC-3' (SEQ. ID. NO. 38, sense)
5'-ctagGGTACCCGGACCTCGCTGGGAGACCTGGAAC-3' (SEQ.ID.NO. 39, antisense).
The sense and anti-sense primers also contained restriction sites for XbaI and KpnI, respectively. These restriction sites are available upstream of the HindIII site in the pcDNA3.1(-) vector.

PCR was then utilized to secure the respective receptor sequences for fusion within the Gsα universal vector disclosed above, using the following protocol for each: 100ng cDNA for GPR3 and GPR6 was added to separate tubes containing 2ul of each primer (sense and anti-sense), 3uL of 10mM dNTPs, 10uL of 10XTaqPlus™ Precision buffer, 1uL of TaqPlus™ Precision polymerase (Stratagene: #600211), and 80uL of water. Reaction temperatures and cycle times for GPR3 were as follows: the initial denaturing step was done it 94°C for five minutes, and a cycle of 94°C for 30 seconds; 55°C for 30 seconds; 72°C for two minutes (repeated 30 times for GPR3). A final extension time was done at 72°C for ten minutes. For GPR6, the initial denaturing step was done at 96°C for seven minutes, and a cycle of 96°C for 30seconds, 55°C for 30 seconds, and 72°C for two minutes was repeated 30 times. A final extension time of ten minutes at 72°C was done for GPR6. Both PCR products for GPR3 and GPR6 were ran on a 1% agarose gel and then purified (data not shown). Each purified product was digested with XbaI and KpnI (New England Biolabs) and the desired inserts were isolated, purified and ligated into the Gs universal vector at the respective restriction site. The positive clones were isolated following transformation and determined by restriction enzyme digest; expression using 293 cells was accomplished following the protocol set forth *infra.* Each positive clone for GPR3:Gs - Fusion Protein and GPR6:Gs - Fusion Protein was sequenced and made available for the direct identification of candidate compounds.

GPCR Fusion Proteins were analyzed as above and verified to be constitutively active (data not shown).

### Example 11

### Protocol: Direct Identification of Inverse Agonists and Agonists Using [³⁵S]GTPγS

Although we have utilized endogenous, constitutively active GPCRs for the direct identification of candidate compounds as, *e*.*g*., inverse agonists, for reasons that are not altogether understood, intra-assay variation can become exacerbated. Preferably, then, a GPCR Fusion Protein, as disclosed above, is utilized. We have determined that when such a protein is used, intra-assay variation appears to be substantially stabilized, whereby an effective signal-to-noise ratio is obtained. This has the beneficial result of allowing for a more robust identification of candidate compounds.

It is important to note that the following results have been obtained using an *orphan* receptor; as that data support, it is possible, using the techniques disclosed herein, to directly identify candidate compounds that modulate the orphan receptor as inverse agonists, agonists and partial agonists, directly from a primary screen; indeed, the methods disclosed herein are sensitive enough to allow for direct identification of both inverse agonist and agonist modulators on the same assay plate.

### 1. Membrane Preparation

Membranes comprising the endogenous, constitutively active orphan GPCR fusion protein of interest (*see* Examples 2 and 10) and for use in the direct identification of candidate compounds as inverse agonists, agonists or partial agonists were prepared as follows:

### (a) Materials

Membrane Scrape Buffer was comprised of 20mM HEPES and 10mM EDTA, pH 7.4; Membrane Wash Buffer was comprised of 20 mM HEPES and 0.1 mM EDTA, pH 7.4; Binding Buffer was comprised of 20mM HEPES, 100 mM NaCl, and 10 mM MgCl₂, pH 7.4

### (b) Procedure

All materials were kept on ice throughout the procedure. Firstly, the media was aspirated from a confluent monolayer of cells, followed by rinse with 10ml cold PBS, followed by aspiration. Thereafter, 5ml of membrane Scrape Buffer was added to scrape cells; this was followed by transfer of cellular extract into 50ml centrifuge tubes (centrifuged at 20,000 rpm for 17 minutes at 4°C). Thereafter, the supernatant was aspirated and the pellet was resuspended in 30ml Membrane Wash Buffer followed by centrifuge at 20,000 rpm for 17 minutes at 4°C. The supernatant was then aspirated and the pellet resuspended in Binding Buffer. This was then homogenized using a Brinkman polytron™ homogenizer (15-20 second bursts until the all material was in suspension). This is referred to herein as "Membrane Protein".

### 2. Bradford Protein Assay

Following the homogenization, protein concentration of the membranes was determined using the Bradford Protein Assay (protein can be diluted to about 1.5mg/ml, aliquoted and frozen (-80°C) for later use; when frozen, protocol for use is as follows: on the day of the assay, frozen Membrane Protein is thawed at room temperature, followed by vortex and then homogenized with a polytron at about 12 x 1,000 rpm for about 5-10 seconds; it is noted that for multiple preparations, the homogenizor should be thoroughly cleaned between homoginezation of different preparations).

### (a) Materials

Binding Buffer (as per above); Bradford Dye Reagent; Bradford Protein Standard were utilized, following manufacturer instructions (Biorad, cat. no. 500-0006).

### (b) Procedure

Duplicate tubes were prepared, one including the membrane, and one as a control "blank". Each contained 800ul Binding Buffer. Thereafter, 10ul of Bradford Protein Standard (1mg/ml) was added to each tube, and 10ul of membrane Protein was then added to just one tube (not the blank). Thereafter, 200ul of Bradford Dye Reagent was added to each tube, followed by vortex of each. After five (5) minutes, the tubes were re-vortexed and the material therein was transferred to cuvettes. The cuvettes were then read using a CECIL 3041 spectrophotometer, at wavelength 595.

### 3. Direct Identification Assay

### (a) Materials

GDP Buffer consisted of 37.5 ml Binding Buffer and 2mg GDP (Sigma, cat. no. G-7127), followed by a series of dilutions in Binding Buffer to obtain 0.2 uM GDP (final concentration of GDP in each well was 0.1 uM GDP); each well comprising a candidate compound, had a final volume of 200ul consisting of 100ul GDP Buffer (final concentration, 0.1uM GDP), 50ul Membrane Protein in Binding Buffer, and 50ul [³⁵S]GTPγS (0.6 nM) in Binding Buffer (2.5 ul [³⁵S]GTPγS per 10ml Binding Buffer).

### (b) Procedure

Candidate compounds (Tripos, Inc., St. Louis, MO) were received in 96-well plates (these can be frozen at -80°C). Membrane Protein (or membranes with expression vector excluding the GPCR Fusion Protein, as control), were homogenized briefly until in suspension. Protein concentration was then determined using the Bradford Protein Assay set forth above. Membrane Protein (and control) was then diluted to 0.25mg/ml in Binding Buffer (final assay concentration, 12.5ug/well). Thereafter, 100 ul GDP Buffer was added to each well of a Wallac Scintistrip™ (Wallac). A 5ul pin-tool was then used to transfer 5 ul of a candidate compound into such well (*i*.*e*., 5ul in total assay volume of 200 ul is a 1:40 ratio such that the final screening concentration of the candidate compound is 10uM). Again, to avoid contamination, after each transfer step the pin tool was rinsed in three reservoirs comprising water (1X), ethanol (1X) and water (2X) - excess liquid should be shaken from the tool after each rinse and dried with paper and kimwipes. Thereafter, 50 ul of Membrane Protein is added to each well (a control well comprising membranes without the GPCR Fusion Protein is also utilized), and pre-incubated for 5-10 minutes at room temperature (the plates were covered with foil in that the candidate compounds obtained from Tripos are light sensitive). Thereafter, 50 ul of [³⁵S]GTPγS (0.6 nM) in Binding Buffer was added to each well, followed by incubation on a shaker for 60 minutes at room temperature (again, in this example, plates were covered with foil). The assay was then stopped by spinning of the plates at 4000 RPM for 15 minutes at 22°C. The plates were then aspirated with an 8 channel manifold and sealed with plate covers. The plates were then read on a Wallacc 1450 using setting "Prot. #37" (as per manufacturer instructions).

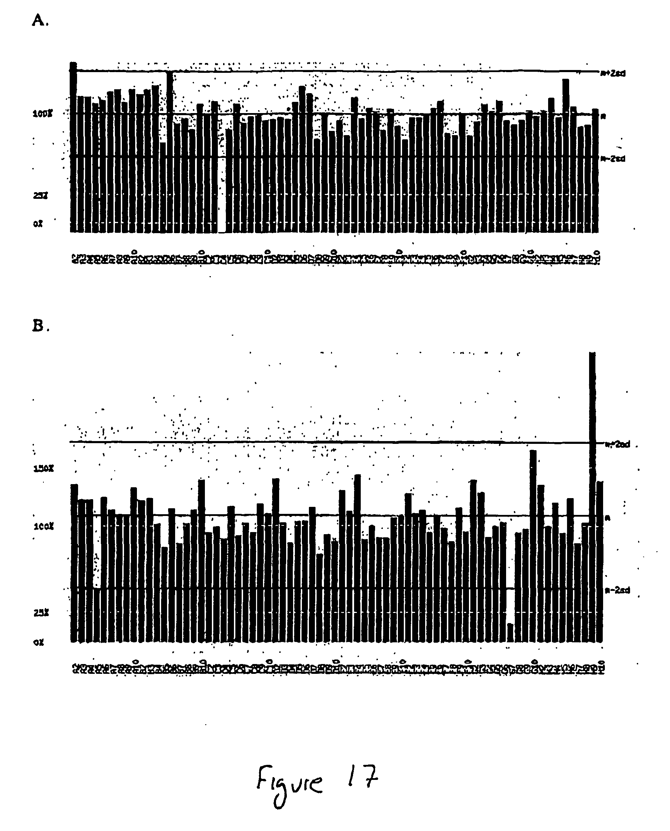

Exemplary results are presented in Figure 17A (GPR3:Gs Fusion Protein) and Figure 17B (GPR6:Gs Fusion Protein) where each designation is a well comprising a different candidate compound, standard deviations based upon the mean results of each plate are in dashed lines and the vertical lines are the percent response. Note in Figure 17A well designation C4 - this compound was directly identified as an inverse agonist to the GPR3 receptor. Note in Figure 17B wells designated G7 and H9 - these compounds were directly identified as an inverse agonist and a agonist, respectively, to the GPR6 receptor. In both cases, these are orphan receptors.

It is preferred that following such direct identification, IC₅₀ (inverse agonist) or EC₅₀ (agonist) values be determined; those having ordinary skill in the art are credited with utilizing IC₅₀ and EC₅₀ assay protocols of choice.

### Example 12

### Protocol: Confirmation Assay

After using an independent assay approach to provide a directly identified candidate compound as set forth above, it is preferred that a confirmation assay then be utilized. In this case, the preferred confirmation assay is a cyclase-based assay.

A modified Flash Plate™ Adenylyl Cyclase kit (New England Nuclear, Cat. No. SMP004A) was utilized for confirmation of candidate compounds directly identified as inverse agonists and agonists to endogenous, constitutively activated orphan GPCRs in accordance with the following protocol.

Transfected cells were harvested approximately three days after transfection. Membranes were prepared by homogenization of suspended cells in buffer containing 20mM HEPES, pH 7.4 and 10mM MgCl₂. Homogenization was performed on ice using a Brinkman Polytron™ for approximately 10 seconds. The resulting homogenate was centrifuged at 49,000 X g for 15 minutes at 4°C. The resulting pellet was then resuspended in buffer containing 20mM HEPES, pH 7.4 and 0.1 mM EDTA, homogenized for 10 seconds, followed by centrifugation at 49,000 X g for 15 minutes at 4°C. The resulting pellet can be stored at -80°C until utilized. On the day of direct identification screening, the membrane pellet is slowly thawed at room temperature, resuspended in buffer containing 20mM HEPES, pH 7.4 and 10mM MgCL2, to yield a final protein concentration of 0.60mg/ml (the resuspended membranes are placed on ice until use).

cAMP standards and Detection Buffer (comprising 2 µCi of tracer [¹²⁵I cAMP (100 µl] to 11 ml Detection Buffer) were prepared and maintained in accordance with the manufacturer's instructions. Assay Buffer was prepared fresh for screening and contained 20mM HEPES, pH 7.4, 10mM MgCl₂, 20mM phospocreatine (Sigma), 0.1 units/ml creatine phosphokinase (Sigma), 50 µM GTP (Sigma), and 0.2 mM ATP (Sigma); Assay Buffer can be stored on ice until utilized.

Candidate compounds identified as per above (if frozen, thawed at room temperature) were added to plate wells (3µl/well; 12µM final assay concentration), together with 40 µl Membrane Protein (30µg/well) and 50µl of Assay Buffer. This admixture was then incubated for 30 minutes at room temperature, with gentle shaking.

Following the incubation, 100µl of Detection Buffer was added to each well followed by incubation for 2-24 hours. Plates were then counted in a Wallac MicroBeta™ plate reader using "Prot. #31" (as per manufacturer instructions).

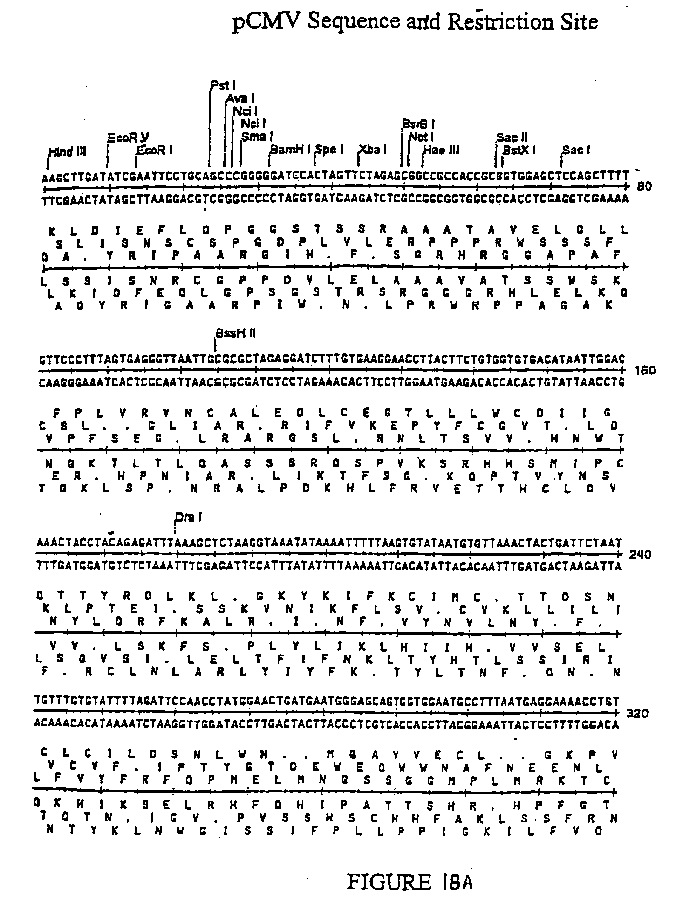

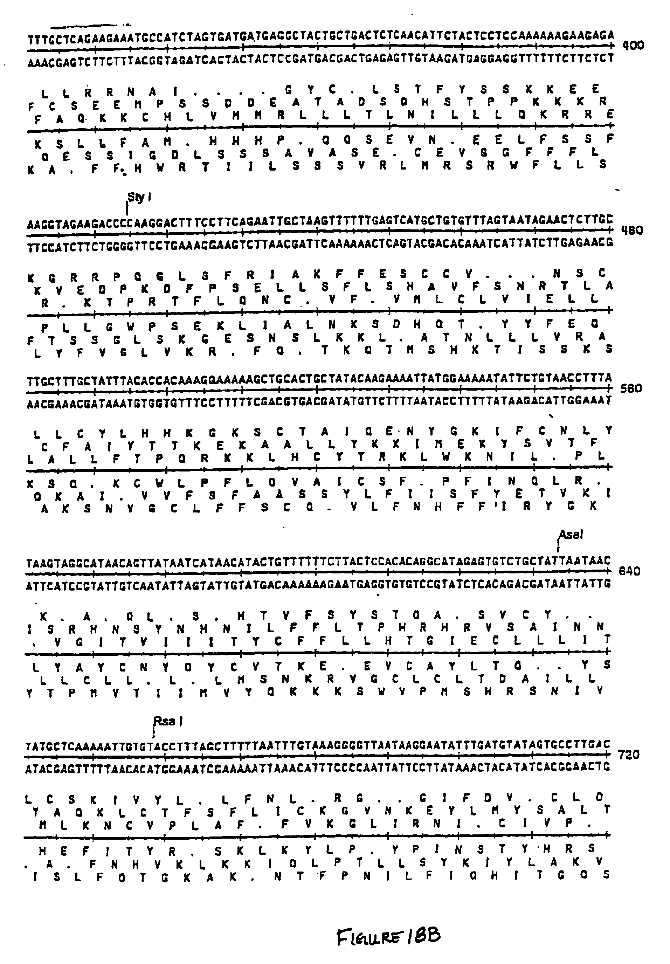

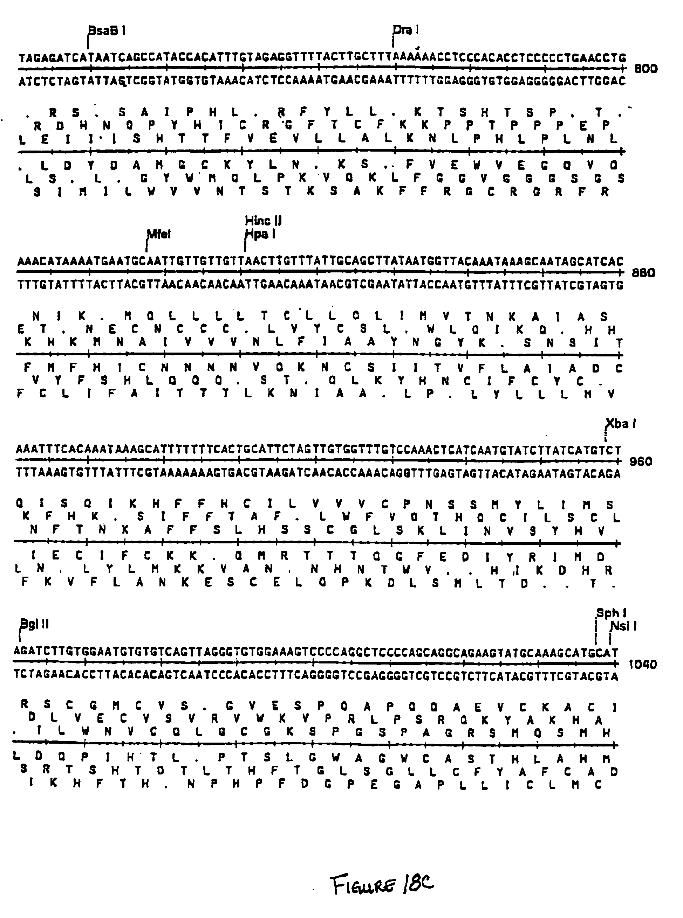

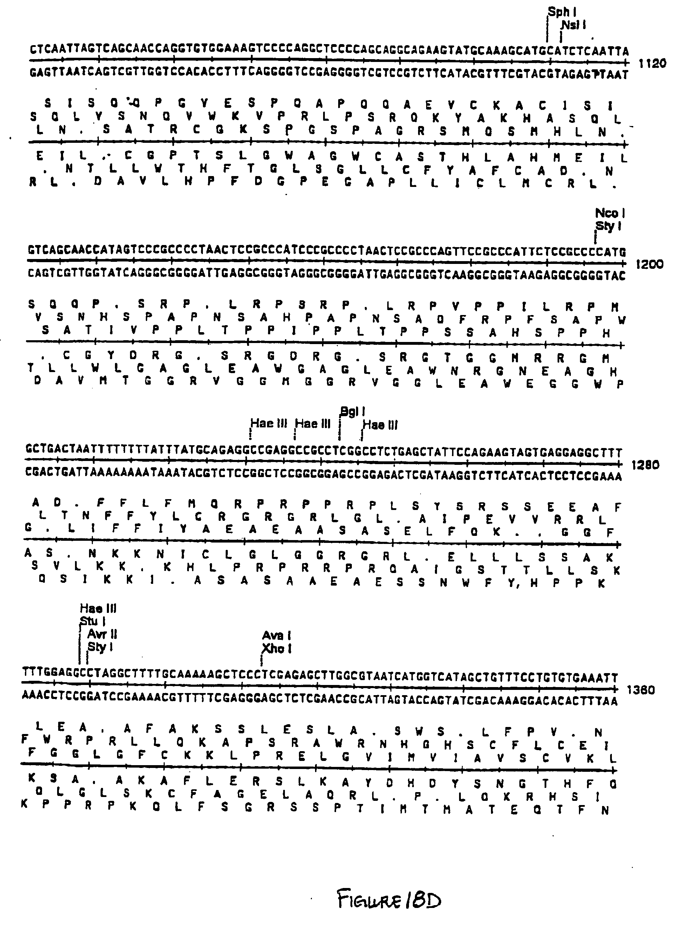

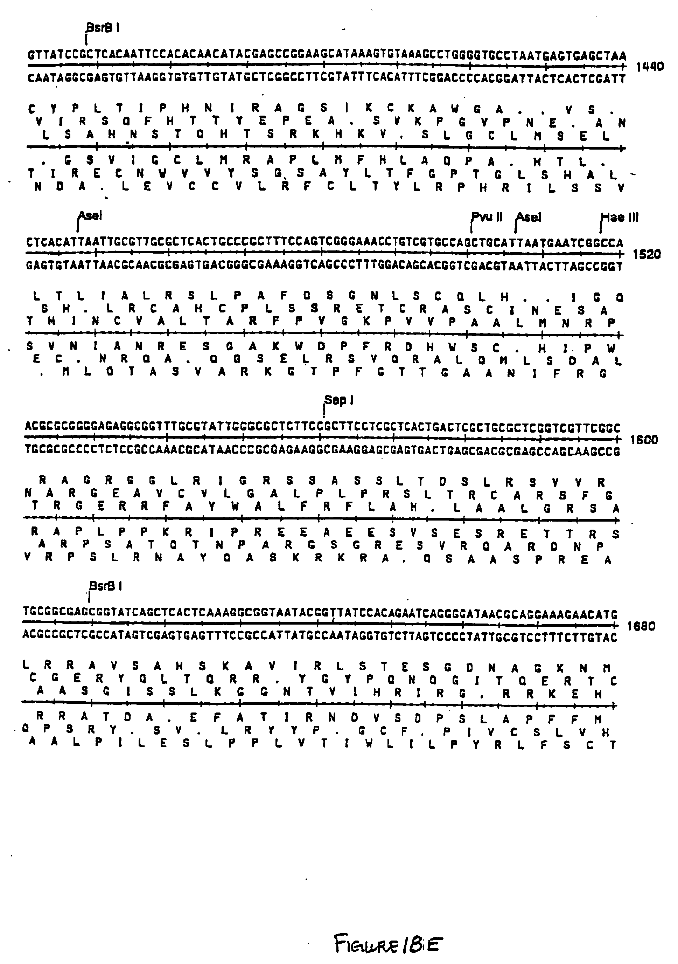

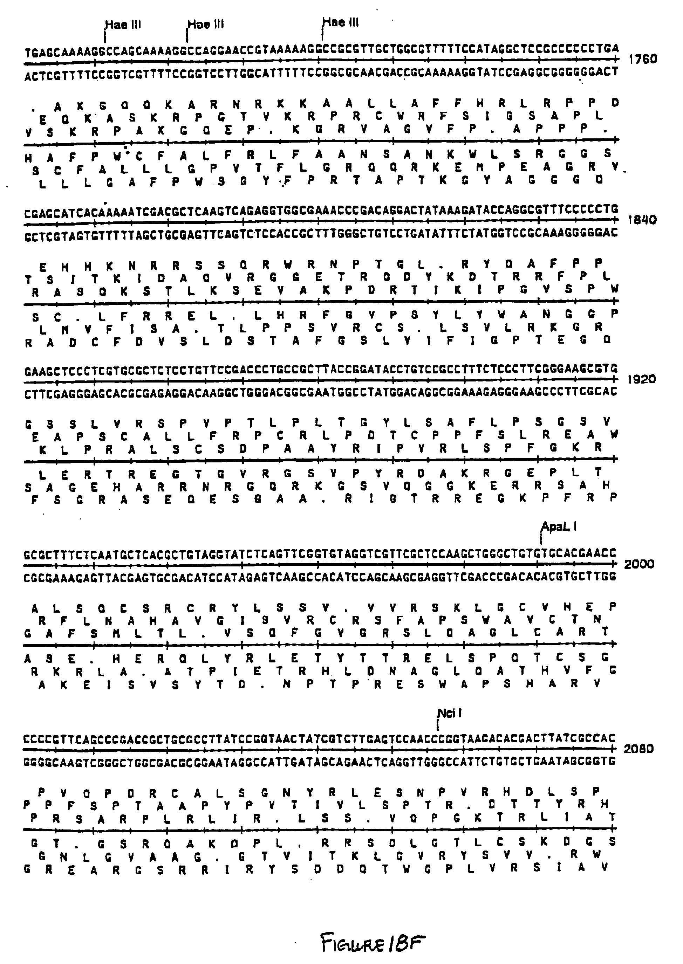

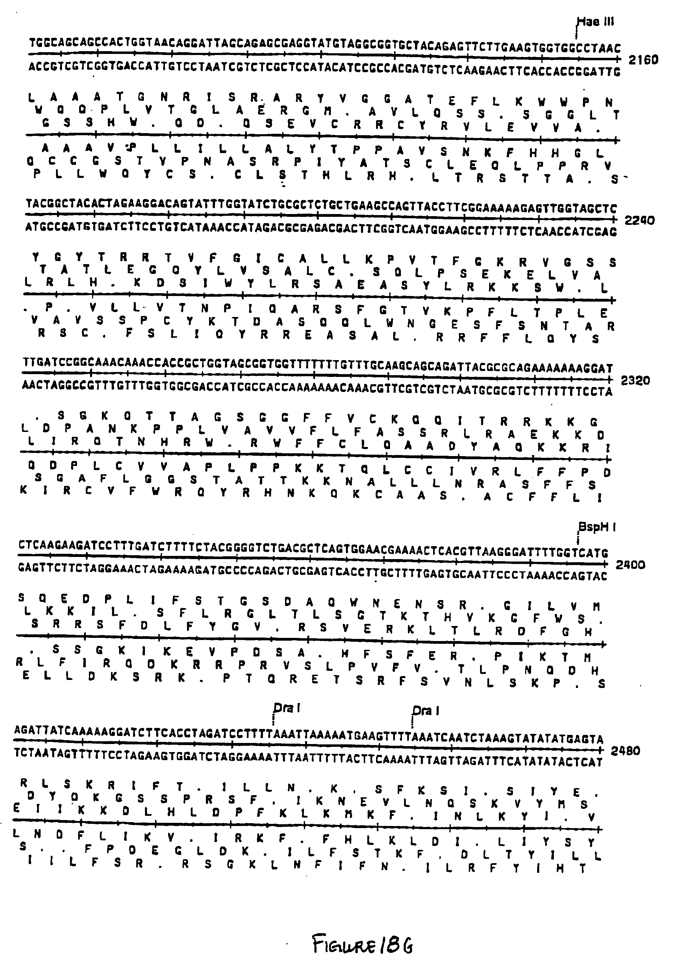

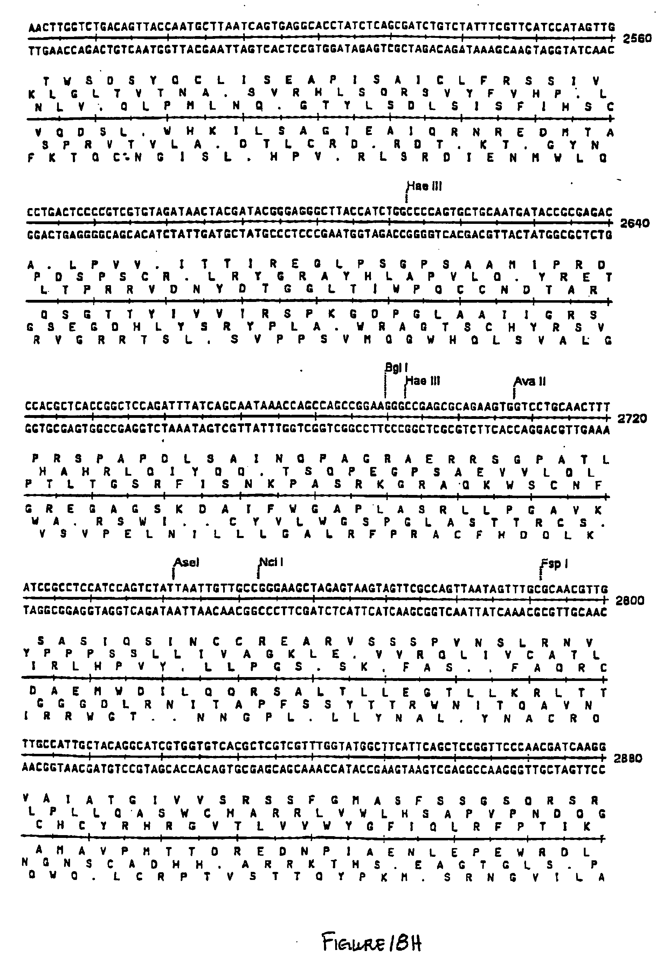

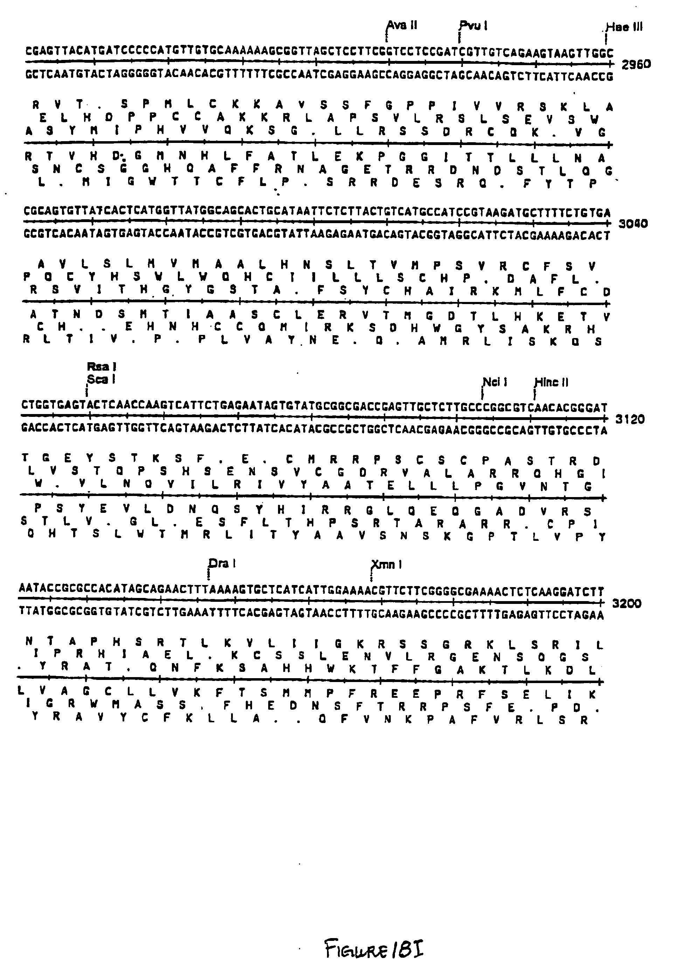

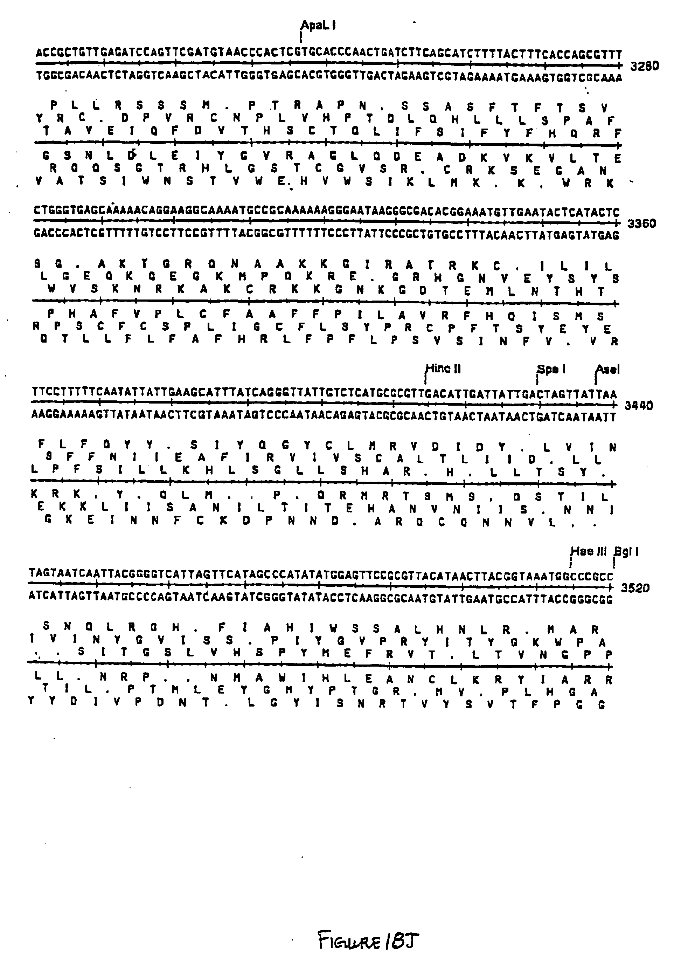

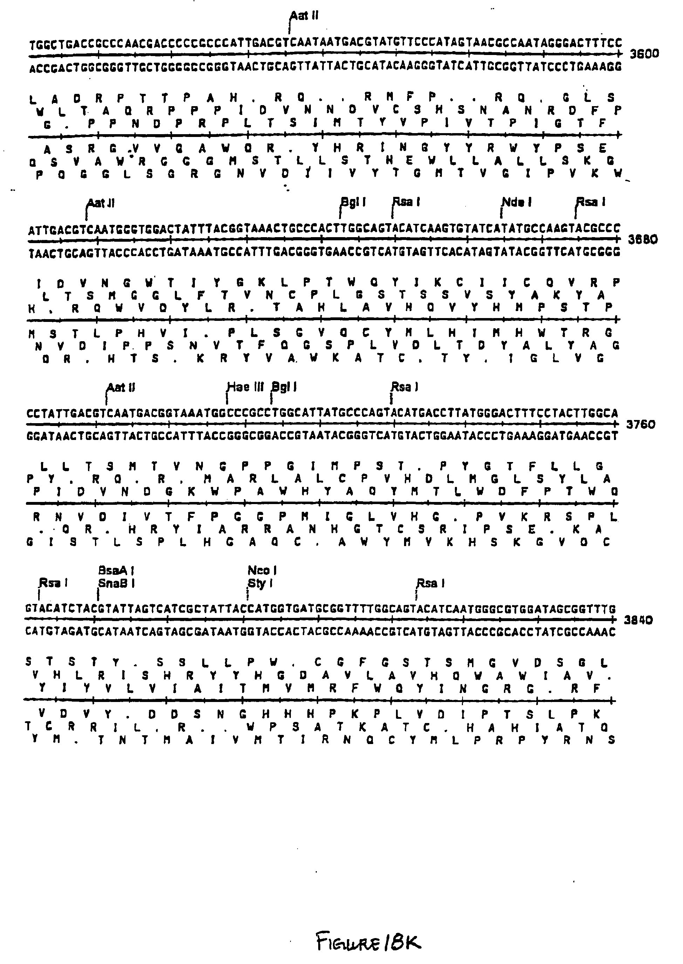

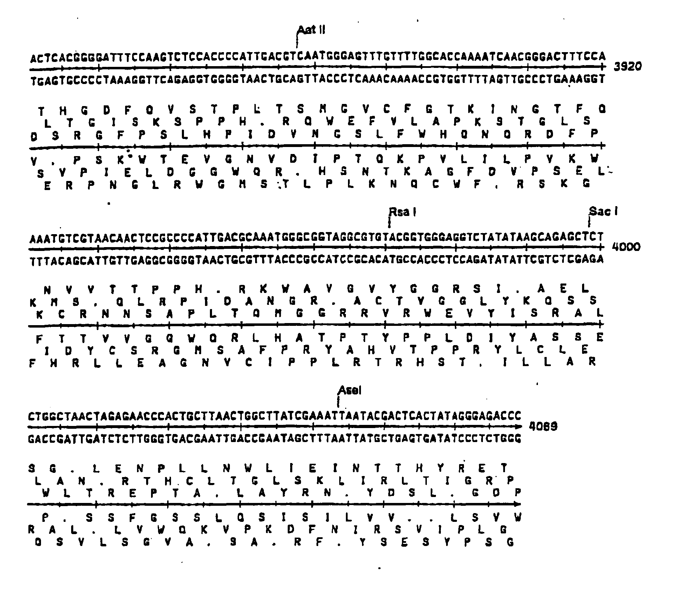

Although a variety of expression vectors are available to those in the art, it is most preferred that the vector utilized be pCMV. This vector has been deposited with the American Type Culture Collection (ATCC) on October 13, 1998 (10801 University Blvd., Manassas, VA 20110-2209 USA) under the provisions of the Budapest Treaty for the International Recognition of the Deposit of Microorganisms for the Purpose of Patent Procedure. The vector was tested by the ATCC and determined to be viable. The ATCC has assigned the following deposit number to pCMV: ATCC #203351. A diagram of pCMV (including restriction sites) is set forth in Figure 18.

As those skilled in the art will appreciate, numerous changes and modifications may be made to the preferred embodiments of the invention, while still remaining within the scope of the invention as defined by the claims.

### Appendix A

### Figure 3 Grid Code

A2 - amygdala; A3 - caudate nucleus; A4 - cerebellum; A5 - cerebral cortex; A6 - frontal cortex; A7 - hippocampus; A8 - medulla oblongata
B1 - occipital cortex; B2 - putamen; B3 - substantia nigra; B4 - temporal cortex; B5 - thalamus; B6 - sub-thalamic nucleus; B7 - spinal cord
C1 - heart; C2 - aorta; C3 - skeletal muscle; C4 - colon; C5 - bladder; C6 - uterus; C7 - prostate; C8 - stomach
D1 - testis; D2 - ovary; D3 - pancreas; D4 - pituitary gland; D5 - adrenal gland; D6 - thyroid; D7 - salivary gland; D8 - mammary gland
E1 - kidney; E2 - liver; E3 - small intestine; E4 - spleen; E5 - thymus; E6 -peripheral leukocyte; E-8 lymph node; E9 - bone marrow
F1 - tonsil; F2 - lung; F3 - trachea; F4 - placenta
G1 - fetal brain; G2 - fetal heart; G3 - fetal kidney; G4 - fetal liver; G5 - fetal spleen; G6 - fetal thymus; G8 - fetal lung

## Claims

1. A method for directly identifying a candidate compound as a compound selected from the group consisting of an inverse agonist, a partial agonist and an agonist, to an endogenous, constitutively active mammalian G protein coupled orphan receptor, comprising the steps of:
(a) contacting a candidate compound with GPCR Fusion Protein expressed in a mammalian cell, said GPCR Fusion Protein comprising an endogenous, constitutively active mammaliam G protein coupled orphan receptor and a G protein; and
(b) determining, by measurement of the ability of the compound to inhibit or stimulate receptor functionality of said contacted receptor, whether said compound is an inverse agonist, a partial agonist or an agonist of said receptor.

2. The method of claim 1 wherein the compound is directly identified as an inverse agonist to said orphan receptor.

3. The method of claim 1 wherein the compound is directly identified as an agonist to said orphan receptor.

4. The method of claim 1 wherein the compound is directly identified as partial agonist to said orphan receptor.

5. The method of claim 1 wherein said orphan receptor is selected from the group consisting of: GPR3, GPR4, GPR6, GPR12, GPR21, OGR1, GHSR, RE2 and ALO22171.

6. The method of claim 1 wherein said orphan receptor is GPR6.

7. The method of claim 1 wherein said G protein is selected from the group consisting of: Gs, Gi, Gq and Go.

8. The method of claim 1 wherein said G protein is Gsα.

9. The method of any one of the preceding claims, wherein said receptor is a human receptor.

## Patentansprüche

1. Verfahren zur direkten Identifizierung einer Kandidatenverbindung als eine aus der aus einem inversen Agonisten, einem partiellen Agonisten und einem Agonisten für einen endogenen, konstitutiv aktiven G-Protein-gebundenen Säugetierwaisenrezeptor bestehenden Gruppe ausgewählte Verbindung, wobei das Verfahren folgende Schritte umfasst:
(a) Kontaktieren einer Kandidatenverbindung mit in einer Säugetierzelle exprimiertem GPCR-Fusionsprotein, wobei das GPCR-Fusionsprotein einen endogenen, konstitutiv aktiven G-Protein-gebundenen Säugetierwaisenrezeptor und ein G-Protein umfasst, und
(b) Bestimmen durch das Messen der Fähigkeit der Verbindung zur Hemmung oder Stimulation der Rezeptorfunktionalität des kontaktierten Rezeptors, ob die Verbindung ein inverser Agonist, ein partieller Agonist oder ein Agonist für den Rezeptor ist.

2. Verfahren nach Anspruch 1, worin die Verbindung direkt als inverser Agonist für den Waisenrezeptor identifiziert wird.

3. Verfahren nach Anspruch 1, worin die Verbindung direkt als Agonist für den Waisenrezeptor identifiziert wird.

4. Verfahren nach Anspruch 1, worin die Verbindung direkt als partieller Agonist für den Waisenrezeptor identifiziert wird.

5. Verfahren nach Anspruch 1, worin der Waisenrezeptor aus der aus GPR3, GPR4, GPR6, GPR12, GPR21, OGR1, GHSR, RE2 und ALO22171 bestehenden Gruppe ausgewählt ist.

6. Verfahren nach Anspruch 1, worin der Waisenrezeptor GPR6 ist.

7. Verfahren nach Anspruch 1, worin das G-Protein aus der aus Gs, Gi, Gq und Go bestehenden Gruppe ausgewählt ist.

8. Verfahren nach Anspruch 1, worin das G-Protein Gsa ist.

9. Verfahren nach einem der vorangegangenen Ansprüche, worin der Rezeptor ein menschlicher Rezeptor ist.

## Revendications

1. Méthode pour l'identification directe d'un composé candidat comme un composé sélectionné dans le groupe consistant en un agoniste inverse, un agoniste partiel et un agoniste, à un récepteur orphelin endogène à activation constitutive couplée à la protéine G mammalienne, comprenant les étapes de:
(a) mettre en contact un composé candidat avec une protéine fusion GPCR exprimée dans une cellule mammalienne, ladite protéine de fusion GPCR comprenant un récepteur orphelin endogène à activation constitutive couplée à la protéine G mammalienne et une protéine G; et
(b) déterminer, par la mesure de l'aptitude du composé à inhiber ou stimuler la fonctionnalité de réception dudit récepteur contacté, si ledit composé est un agoniste inverse, un agoniste partiel ou un agoniste dudit récepteur.

2. Méthode selon la revendication 1, dans laquelle le composé est directement identifié comme un agoniste inverse audit récepteur orphelin.

3. Méthode selon la revendication 1, dans laquelle le composé est directement identifié comme un agoniste audit récepteur orphelin.

4. Méthode selon la revendication 1, dans laquelle le composé est directement identifié comme un agoniste partiel audit récepteur orphelin.

5. Méthode selon la revendication 1, dans laquelle ledit récepteur orphelin est sélectionné dans le groupe consistant en: GPR3, GPR4, GPR6, GPR12, GPR21, OGR1, GHSR, RE2 et ALO22171.

6. Méthode selon la revendication 1, dans laquelle ledit récepteur orphelin est GPR6.

7. Méthode selon la revendication 1, dans laquelle ladite protéine G est sélectionnée dans le groupe consistant en: Gs, Gi, Gq et Go.

8. Méthode selon la revendication 1, dans laquelle ladite protéine G est G_{Sα}.

9. Méthode selon l'une quelconque des revendications précédentes, dans laquelle ledit récepteur est un récepteur humain.
